(19) **Europäisches Patentamt**
**European Patent Office**
**Office européen des brevets**

(11) **EP 4 049 586 A1**

(12) **EUROPEAN PATENT APPLICATION**

(43) Date of publication:
**31.08.2022 Bulletin 2022/35**

(21) Application number: **21159658.0**

(22) Date of filing: **26.02.2021**

(51) International Patent Classification (IPC):
*A61B 5/145* (2006.01)    *A61B 5/1486* (2006.01)
*A61B 5/1473* (2006.01)    *G01N 27/327* (2006.01)
*A61B 5/00* (2006.01)

(52) Cooperative Patent Classification (CPC):
**A61B 5/14532; A61B 5/14507; A61B 5/14546;**
**A61B 5/14735; A61B 5/1486; A61B 5/7271;**
**G01N 27/3271**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(71) Applicants:
• **Roche Diabetes Care GmbH**
  **68305 Mannheim (DE)**
  Designated Contracting States:
  **DE**
• **F. Hoffmann-La Roche AG**
  **4070 Basel (CH)**
  Designated Contracting States:
  **AL AT BE BG CH CY CZ DK EE ES FI FR GB GR**
  **HR HU IE IS IT LI LT LU LV MC MK MT NL NO PL**
  **PT RO RS SE SI SK SM TR**

(72) Inventors:
• **SLIOZBERG, Kirill**
  **68305 Mannheim (DE)**
• **WEHOWSKI, Frederic**
  **68305 Mannheim (DE)**

(74) Representative: **Altmann Stößel Dick**
**Patentanwälte PartG mbB**
**Theodor-Heuss-Anlage 2**
**68165 Mannheim (DE)**

(54) **METHOD FOR DETERMINING A MEMBRANE PROPERTY OF AN ANALYTE SENSOR**

(57)    A method for determining at least one membrane property of an analyte sensor (112) is proposed. The analyte sensor (112) comprises at least two measurement electrodes (114). At least one of the measurement electrodes (114) comprises at least one membrane element (122) having at least one membrane property. The method comprising the following steps:
a) (134) generating at least one fast-transient voltage signal and applying the fast-transient voltage signal to the measurement electrodes (114) at an application time to;
b) (136) measuring a first response signal $U_1$ at a first time $t_1$ and a second response signal $U_2$ at a second time $t_2$ with $t_0 \neq t_1 \neq t_2$, wherein the application time $t_0$ precedes the first time $t_1$ and the second time $t_2$;
c) (138) determining a response signal $U_0$ at the application time $t_0$ by evaluating the first response signal $U_1$ and the second response signal $U_2$;
d) (140) determining the at least one membrane property by evaluating of the response signal $U_0$ at the application time $t_0$.

Fig. 3

**EP 4 049 586 A1**

## Description

Field of the invention

The present invention discloses a method for determining at least one membrane property of an analyte sensor, a method for determining a concentration of at least one analyte in bodily fluid using at least one analyte sensor and an analytical system. The analyte sensor may be or may comprise an electrochemical sensor configured for insertion into a bodily tissue of a user, specifically an insertable or implantable electrochemical sensor for monitoring of the at least one analyte in the bodily tissue and/or in a bodily fluid within the bodily tissue. The method and devices according to the present invention may be used for detecting at least one analyte present in one or both of a bodily tissue or a bodily fluid, in particular the method and devices are applied in the field of detecting one or more analytes such as glucose, lactate, triglycerides, cholesterol or other analytes, e.g. metabolites, in bodily fluids such as blood or interstitial fluid or other bodily fluids, both in the field of professional diagnostics, in the field of hospital point of care, in the field of personal care and in the field of home monitoring. However, other fields of application are feasible.

Related art

In the field of medical technology and diagnostics, a large number of devices and methods for detecting at least one analyte in a bodily fluid are known. The method and devices may be used for detecting at least one analyte present in one or both of a bodily tissue or a bodily fluid, in particular one or more metabolites, in particular one or more analytes such as glucose, lactate, triglycerides, cholesterol or other analytes in bodily fluids such as blood or interstitial fluid or other bodily fluids. Without restricting the scope of the present invention, in the following, mainly reference is made to the determination of glucose by an electrochemical biosensor as an exemplary and preferred analyte.

A typical electrochemical biosensor comprises a biological recognition element, which can be an antibody, a DNA-string, a protein or more specifically an enzyme. These molecules specifically bind to or react with analyte molecules. The biological recognition element, here exemplary an enzyme, is in contact to a transducer, an element, which transforms the change in the biological recognition element in to a measurable signal. Typical electrochemical biosensor uses working electrode as a transducer. In the case of enzymatic electrodes, the charge (electrons) generated by the enzyme must be efficiently and/or quantitatively collected by the transducer. Depending on the used enzyme and the sensor construction, the charge transfer can be direct from the enzyme to the transducer, i.e. the working electrode, or redox mediated by e.g. natural oxygen, redox-active polymers or other redox active substances. The here exemplary presented electrochemical sensor deploys the enzyme from the class of oxidoreductase, called glucose oxidase (GOx). GOx may use oxygen as an electron acceptor, reducing it to hydrogen peroxide. The latter is diffusing toward working electrode surface, which is polarised at a potential, sufficient for efficient oxidation of the hydrogen peroxide. Thus, the oxygen/hydrogen peroxide acts as redox mediator for electron transfer from the enzyme active center to the surface of the working electrode. Such scheme corresponds to an enzymatic biosensor of the first generation. In the second generation, other redox reagents are envisaged to replace oxygen. Such mediators may be either freely diffusing species, or bound in a polymer matrix or other way. Some examples of the redox active species are ferrocene and phenazine derivatives, quinones, ruthenium complexes or osmium complexes.

In the field of continuous monitoring, typically, subcutaneous implantable electrochemical sensors are used. A typical subcutaneous continuous glucose sensor is based on an enzymatic oxidation of glucose, which is present in the interstitial fluid (ISF). Glucose concentration in the ISF of the skin is relatively high which may lead to the following problems.

1. The oxidation kinetics of the enzyme may be limiting. Typically, enzymes have such characteristic as turnover number (TON), the maximum number of chemical conversions of molecules, e.g. glucose, per second that a single catalytic site will execute for a given enzyme concentration. It may not be possible for the enzyme to oxidize large amount of glucose such that the enzyme may be the limiting factor of the measuring chain and makes a quantitative measurement impossible.

2. The lifetime under load may be limiting. The turnover number may also have a different meaning, the number of moles of substrate, e.g. glucose, that a mole of catalyst, here the enzyme, can convert before becoming, fully or partially, e.g. to one half of the initial activity, inactivated. Thus, under this high utilization the enzymatic electrode may rapidly lose activity.

3. If the amount of enzyme is enough to oxidize a large amount of glucose, other factors may be limiting, such that a quantitative measurement is impossible. For instance, the kinetics of the electron transfer from the enzyme to the transducer may be the limiting factor.

4. If the activity of the enzymatic electrodes may be adjusted such that the high concentration of glucose is efficiently oxidized and the electron transfer to the electrode is efficient, a local depletion of glucose may exist. Glucose may

2

diffuse relatively slowly in the ISF such that the concentration of glucose in the region of the sensor where it is actively consumed, may be lower compared to the ISF such that a correct and quantitative measurement is not possible.

5. An electrochemical continuous glucose sensor may comprise at least two electrodes, wherein onto one of the electrodes, here denoted as working electrode, the glucose detection by means of oxidation chain happens. A second electrode, denoted counter or auxiliary electrode, is used in order to complete the electrochemical process and to provide a counter reaction to compensate the charge flow. At the working electrode oxidizing processes occur and at the counter electrode reductive processes occur, wherein the amount of charge must be identical and the counterreaction may not be limiting. In the case, if the counter/auxiliary electrode is subcutaneous as well and is made of an electrochemically inactive substance, e.g. gold, the substance which is reduced at the counter electrode is typically the in ISF dissolved molecular oxygen. However, the amount of available dissolved oxygen is significantly less than that of glucose such that the counter reaction may be limiting and a quantitative measurement is thus impossible.

A solution for the aforementioned problems may be using a so-called diffusion limiting layer. The layer may be applied to the working electrode as thin polymer film forming a membrane and may be configured for slowing down the diffusion of the glucose to the sensitive surface of the working electrode. Thus, the glucose concentration directly at the sensitive surface of the working electrode is less but proportional to the glucose concentration in the ISF. However, in order to allow for a correct quantitative measurement of the glucose concentration permeability of the membrane needs to be constant or known. The direct measurement of the membrane permeability *in vivo* is not possible or very challenging, in particular in case no other nominal values are known from which the permeability can be determined.

Moreover, the permeability of the membrane may depend on several factors such as the material of the membrane, thickness of the membrane, temperature, swelling degree and others. In known methods, impact on temperature may be determined using an external temperature sensor which is placed on the skin. However, as the temperature is determined on the skin but not subcutaneous at a position of the sensor, reliability and accuracy of these methods may be limited.

Several electrochemical methods are known for compensating membrane effects such as using electrochemical impedance spectroscopy or potential pulse techniques. However, these methods may require complex electronics. Moreover, conducting of these additional measurements may result in driving the electrochemical system out of its steady-state, thus the correct measurement during this time and, maybe, sometime after, is not possible. In addition, applying of the additional modulation potential may provoke side effects, such as unspecific oxidation of interference substances which may lead to a non-correct measurement values.

Furthermore, these methods are not always sufficiently specific towards membrane effects, and may be influenced by other parameters of the system, such as actual analyte concentration and thus actual signal level, e.g. DC current.

US 2010/0213079 A1 describes a system for the measurement of analyte concentration which includes an electrochemical cell having a working electrode coated with a protein layer and a diffusion limiting barrier covering the protein layer, and a counter electrode; a voltage source which provides a voltage between the working electrode and the counter electrode when electrically connected by a conductive medium; and a computing system which measures the dynamic voltage output to the counter electrode within a time period prior to a response from the working electrode and method for use is disclosed.

WO 2019/115687 A1 describes a method for determining an information on an equivalent series resistance in a test strip

EP application number 20 162 098.6 filed on March 10, 2020, the full content of which is included by reference, describes a method for determining a membrane property by applying a fast transient voltage signal and measuring the response signal to obtain information on membrane properties.

Problem to be solved

It is therefore an objective of the present invention to provide a method for determining at least one membrane property of an analyte sensor, a method for determining a concentration of at least one analyte in bodily fluid using at least one analyte sensor and an analytical system, which at least partially avoid the shortcomings of known devices and methods of this kind and which at least partially address the above-mentioned challenges. Specifically, a method for determining permeability of a membrane with reduced complexity and enhanced reliability shall be provided.

Summary

This problem is solved by a method for determining at least one membrane property of an analyte sensor, a method for determining a concentration of at least one analyte in bodily fluid using at least one analyte sensor, and an analytical system, with the features of the independent claims. Preferred embodiments, which might be realized in an isolated fashion or in any arbitrary combination, are listed in the dependent claims and throughout the specification.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, it shall be noted that the terms "at least one", "one or more" or similar expressions indicating that a feature or element may be present once or more than once typically will be used only once when introducing the respective feature or element. In the following, in most cases, when referring to the respective feature or element, the expressions "at least one" or "one or more" will not be repeated, non-withstanding the fact that the respective feature or element may be present once or more than once.

Further, as used in the following, the terms "preferably", "more preferably", "particularly", "more particularly", "specifically", "more specifically" or similar terms are used in conjunction with optional features, without restricting alternative possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding alternative embodiments of the invention, without any restrictions regarding the scope of the invention and without any restriction regarding the possibility of combining the features introduced in such way with other optional or non-optional features of the invention.

In a first aspect of the present invention, a method for determining at least one membrane property of an analyte sensor is disclosed.

The term "analyte" as used herein is a broad term and is to be given its ordinary and customary meaning to a person of ordinary skill in the art and is not to be limited to a special or customized meaning. The term specifically may refer, without limitation, to an arbitrary element, component or compound which may be present in a bodily fluid and the concentration of which may be of interest for a user. Specifically, the analyte may be or may comprise an arbitrary chemical substance or chemical compound which may take part in the metabolism of the user, such as at least one metabolite. As an example, the at least one analyte may be selected from the group consisting of glucose, cholesterol, triglycerides, lactate. Additionally or alternatively, however, other types of analytes may be determined and/or any combination of analytes may be determined.

The term "sensor" as used herein is a broad term and is to be given its ordinary and customary meaning to a person of ordinary skill in the art and is not to be limited to a special or customized meaning. The term specifically may refer, without limitation, to an arbitrary element or device configured for detecting at least one condition or for measuring at least one measurement variable. The term "analyte sensor" as used herein is a broad term and is to be given its ordinary and customary meaning to a person of ordinary skill in the art and is not to be limited to a special or customized meaning. The term specifically may refer, without limitation, to a sensor configured for detecting quantitatively or qualitative at least one analyte. The analyte sensor may be or may comprise at least one electrochemical sensor. The term "electrochemical sensor" specifically may refer to a sensor based on electrochemical measurement principles, such as by using one or more of an amperometric, coulometric or a potentiometric measurement principle. Specifically, the electrochemical sensor may comprise at least one enzyme configured for performing at least one redox reaction in the presence of the analyte to be detected, wherein the redox reaction may be detected by electrical means. As used herein, the term "electrochemical detection" refers to a detection of an electrochemically detectable property of the analyte by electrochemical means, such as an electrochemical detection reaction. Thus, for example, the electrochemical detection reaction may be detected by comparing one or more electrode potentials, such as a potential of a working electrode with the potential of one or more further electrodes such as a counter electrode or a reference electrode. The detection may be analyte specific. The detection may be a qualitative and/or a quantitative detection.

In an embodiment, the sensor may be an optical sensor. The term optical sensor specifically may refer to a sensor based on optical measurement techniques, such as light.

The analyte sensor may be an in-vivo sensor. The term "in-vivo sensor" as used herein is a broad term and is to be given its ordinary and customary meaning to a person of ordinary skill in the art and is not to be limited to a special or customized meaning. The term specifically may refer, without limitation, to a sensor which is configured for being at least partially implanted into a body tissue of a user. The analyte sensor may be a subcutaneous analyte sensor. The analyte sensor may be configured for implantation into a body tissue of the user. More specifically the analyte sensor may be configured for continuous monitoring of the analyte. The analyte sensor may be fully implantable or partially implantable. The term "user" as used herein is a broad term and is to be given its ordinary and customary meaning to a person of ordinary skill in the art and is not to be limited to a special or customized meaning. The term specifically may refer, without limitation, to a human being or an animal, independent from the fact that the human being or animal, respectively, may be in a healthy condition or may suffer from one or more diseases. As an example, the user may be a human being or an animal suffering from diabetes. However, additionally or alternatively, the invention may be applied to other types of users.

The analyte sensor comprises at least two measurement electrodes. The term "measurement electrode" as used herein is a broad term and is to be given its ordinary and customary meaning to a person of ordinary skill in the art and is not to be limited to a special or customized meaning. The term specifically may refer, without limitation, to an electrode which is or can be brought in contact with an electrolyte, in particular with a bodily fluid. The at least two measurement electrodes may be designed such that an electrochemical reaction may take place at one or more of the electrodes. Thus, the measurement electrodes may be embodied such that an oxidation reaction and/or reduction reaction may take place at one or more of the electrodes.

One of the measurement electrodes may be designed as working electrode. The term "working electrode" as used herein is a broad term and is to be given its ordinary and customary meaning to a person of ordinary skill in the art and is not to be limited to a special or customized meaning. The term specifically may refer, without limitation, to an electrode of the analyte sensor which is configured for measuring a signal, such as a voltage, a current, a charge or electrical/electrochemical potential, dependent on the degree of an electrochemical detection reaction taking place at the working electrode, for the purpose of detecting the at least one analyte. The working electrode may comprise at least one test chemical. The working electrode may fully or partially be covered with at least one test chemical, specifically at least one test chemical comprising at least one enzyme for detecting the at least one analyte. As an example, glucose oxidase (GOx) or glucose dehydrogenase (GDH) may be used. The test chemical, further, may comprise additional materials, such as binder materials, electrode particles, mediators or the like. Thus, as an example, the test chemical may comprise at least one enzyme, carbon particles, a polymer binder and $MnO_2$ particles. In another preferred embodiment, the test chemical may comprise a mediator polymer comprising a polymeric material and a metal containing complex, for example a modified poly(vinylpyridine) backbone loaded with poly(bi-imidizyl) Os complexes covalently coupled through a bidentate linkage. Further, the at least one test chemical may be comprised in a single layer, or the test chemical may comprise a plurality of layers, such as one layer having the at least one enzyme and one or more additional layers having one or more additional functions, such as one or more diffusion barriers and/or one or more biocompatibility layers.

The other one of the measurement electrodes may be designed as counter or auxiliary electrode. The term "counter electrode" as used herein is a broad term and is to be given its ordinary and customary meaning to a person of ordinary skill in the art and is not to be limited to a special or customized meaning. The term specifically may refer, without limitation, to an electrode adapted for performing at least one electrochemical counter reaction and/or configured for balancing a current flow due to the detection reaction at the working electrode. The counter electrode may be a part of the implanted or partially implanted analyte sensor, or may be an individual electrode, which is either implanted or partially implanted or placed somewhere else on the body, e.g. on the skin surface. In case of the analyte sensor comprises a two-electrode system as measurement electrodes, the counter electrode may complete the circuit such that charge can flow through an electrochemical cell, also denoted electrochemical system, given by the working electrode, the counter electrode and an electrolyte, such as the bodily fluid, and may maintain a constant counter electrode potential, also referred to as a constant reference potential, regardless of current.

Additionally, the analyte sensor may comprise at least one reference electrode. The term "reference electrode", also referred to as "pseudo reference electrode", specifically may refer, without limitation, to an electrode of the analyte sensor which is configured to provide an electrochemical reference potential which, at least widely, is independent of the presence or absence or concentration of the analyte. The reference electrode may be configured for being a reference for measuring and/or controlling a potential of the working electrode. The reference electrode may have a stable and well-known electrode potential. The electrode potential of the reference electrode may preferably be highly stable. One of the electrodes may have several functionalities, as for instance, combined reference and counter electrode, which has both, the function of the reference and counter electrodes, which means it provides a reference potential and balances the current flow from the working electrode.

At least one of the measurement electrodes comprises at least one membrane element having the at least one membrane property. Specifically, the membrane element may be applied to the working electrode. The term "membrane element" as used herein is a broad term and is to be given its ordinary and customary meaning to a person of ordinary skill in the art and is not to be limited to a special or customized meaning. The term specifically may refer, without limitation, to at least one element configured for controlling and/or limiting diffusion of the analyte to the electrode to which the membrane element is applied. Thus, the membrane element may be configured as diffusion limiting membrane. However, the membrane element may have even more functionalities, such as providing biocompatibility. The membrane element may have further functions such as blocking of leakage of components below the membrane element such as of the enzyme or other components comprised in any one of the at least two measurement electrodes. The membrane element may also be configured as a blocking membrane. As used herein, the term "blocking" may refer to preventing leakage of inner components of a sensitive layer of the working electrode but not to the analyte. The membrane element may be configured for maintaining of sensor integrity, by for instance keeping the enzyme or redox mediator from leaching, thus degradation of the whole sensor. Independently on the role of the membrane element, its altering may be compensated.

The membrane element may comprise at least one polymer. The membrane element may be applied to the working electrode as thin polymer film. For example, the membrane element may be or may comprise Poly-(4-(N-(3-sulfonatopropyl) pyridinium)-co-(4vinyl-pyridine)-co- styrene (5%/90%/5%) or hydrophilic Polyurethane (HP60D20), for example available from Lubrizol®. For example, the membrane element may comprise at least one of the following polymer classes and/or their copolymer: Poly(4 vinyl pyridine), Polymethacrylate, Polyacrylate, Polyvinyl pyrrolidone, Polyvinyl alcohol (PVA), Polyethylene glycol.

The term "membrane property" as used herein is a broad term and is to be given its ordinary and customary meaning to a person of ordinary skill in the art and is not to be limited to a special or customized meaning. The term specifically may refer, without limitation, to an arbitrary physical property of the membrane element influencing the determining of the analyte. Specifically, the membrane property may be permeability of the membrane element. The term "permeability" as used herein is a broad term and is to be given its ordinary and customary meaning to a person of ordinary skill in the art and is not to be limited to a special or customized meaning. The term specifically may refer, without limitation, to a material parameter characterizing transmission properties of the membrane element, specifically passing of substances through the membrane element. Further specifically, permeability may refer to permeability for a specific analyte since molecules and ions of the analytes may have different sizes, shapes and charge. In an embodiment, the permeability refers to the permeability of the membrane for glucose.

Permeability of the membrane element for certain compounds may be proportional to the membrane's swelling degree. The swelling degree may correspond to the degree of water uptake. The swelling degree of the membrane may depend on its hydrophilicity. The membrane's swelling degree may directly affect the amount and/or mobility and, thus, the permeability of the membrane for certain compounds. The conductivity of an electrolyte like water or bodily fluid, such as interstitial fluid is directly linked to so-called total dissolved solids whereby ions, such as H+, OH-, Na+, K+, Cl- and other have the most contribution. Therefore, also the conductivity of the membrane which has taken up water or bodily fluid such as interstitial fluid is directly linked to the total dissolved solids. The more charge carriers are present and the more mobile they are, the lower is the measured electrical resistance, by otherwise constant conditions, such as e.g. cell geometry. Thus, the electrical resistance, or reversely, electric conductivity of the membrane element may depend on quantity and mobility of ions present in the membrane.

The proposed method may comprise using at least one algorithm configured for determining permeability of the membrane element for a specific analyte, in particular glucose, by evaluating electrical resistance of the membrane element. The permeability of the membrane element for a specific analyte $p_{Analyt}$ may be determined by $p_{Analyt} = f*p$, wherein p is the permeability determined via the electrical resistance of the membrane element and f is a conversion factor. The conversion factor may be determined in calibration experiments using known glucose values. The membrane property, in particular the permeability, may depend on different parameters such as temperature, composition of interstitial fluid, thickness of the membrane element, aging, swelling degree, mechanical stresses and the like.

After insertion of the analyte sensor, the membrane element may swell. In ideal case, the swelling process may be rapid such that a determining of the concentration of the analyte is not influenced, or a swelling behavior may be pre-known such that changes in permeability can be considered and corrected. However, in non-ideal case, the swelling of the membrane element may lead to unknown changes in permeability.

Composition of the interstitial fluid may vary from user to user. Components of the interstitial fluid may change permeability of the membrane element such that molecules and ions can ingress from the interstitial fluid into the membrane element. The molecules and ions can bind to certain functional groups of the polymer of the membrane element and can change permeability of the membrane element. Effects due to non-constant interstitial fluid can be temporal, i.e. binding of ingressed molecules and ions to functional groups of the polymer of the membrane element may be reversible. However, even in non-permanent changes diffusion of ingressed molecules and ions out of the membrane may last some time.

Permeability of the membrane element may depend on temperature, as it directly influences the ions mobility within the membrane. The temperature at insertion site of the analyte sensor may not be constant such that in-operando monitoring of permeability may be performed. Intrinsic properties of the membrane element may change during storage of the analyte sensor. These changes may depend on storage conditions. For example, the membrane property may change faster at high temperatures. Such changes may lead to changes in permeability and may lead to non-reliable measurements.

Further, mechanical load may change permeability of the membrane. For example, if a user lays down to bed on a side where the inserted analyte sensor is arranged, skin of the user and the analyte sensor may be mechanically compressed which may result in decrease of the sensor signal.

The partially or fully implanted analyte sensor may comprise at least one biocompatibility layer such as a thin layer of highly hydrophilic polymer. This layer may be applied independently on the presence of the diffusion limiting membrane and may influence the diffusion of the analyte, thus acting as kind of diffusion limiting membrane. For accurate measurements, this effect may be considered and the method according to the present invention may be applied for compensation of biocompatible layers or other layers, which are not deliberately diffusion limiting layers.

The determining of the membrane property may comprise testing the membrane property. The method may further comprise at least one calibration step, wherein effects of the different parameters on the permeability of the membrane element may be determined. For each of the parameters influencing permeability of the membrane element at least one correction factor may be determined by calibration experiments. The method may comprise determining correction factors for interdependent parameters. The method may comprise determining permeability of the membrane element considering the at least one correction factor. The method may comprise in-operando monitoring of permeability, in particular continuously or in short time intervals. Also temperature monitoring is possible. As will be outlined in detail below, the method may comprise at least one failsafe step in order to enhance reliability of the determining of the analyte concentration.

The method comprises the method steps as given in the corresponding independent claim and as listed as follows. The method steps may be performed in the given order. One or more of the method steps may be performed in parallel and/or in a time overlapping fashion. Further, one or more of the method steps may be performed repeatedly. Further, additional method steps may be present which are not listed.

The method comprising the following steps:

a) generating at least one fast-transient voltage signal and applying the fast-transient voltage signal to the measurement electrodes at an application time $t_0$;

b) measuring a first response signal $U_1$ at a first time $t_1$ and a second response signal $U_2$ at a second time $t_2$ with to $\neq t_1 \neq t_2$, wherein the application time $t_0$ precedes the first time $t_1$ and the second time $t_2$;

c) determining a response signal $U_0$ at the application time $t_0$ by evaluating the first response signal $U_1$ and the second response signal $U_2$;

d) determining the at least one membrane property by evaluating of the response signal $U_0$ at the application time $t_0$.

The determining of the membrane property according to the present invention may comprise determining the membrane property using a fast-transient technique as described in EP application number 20 162 098.6 filed on March 10, 2020, the full content of which is included by reference. In particular, the method may comprise generating at least one fast-transient voltage signal and applying the fast-transient voltage signal to the measurement electrodes, measuring a response signal and determining the at least one membrane property by evaluating of the response signal. The evaluating of the response signal may comprise determining equivalent series resistance of the analyte sensor and determining the at least one membrane property from the equivalent series resistance of the analyte sensor. The unknown equivalent series resistance to be determined may be serially connected with a known reference resistor. The reference resistor may have a value roughly matching the range of the unknown resistance, as will be described in more detail below. A signal generator device may apply a short voltage pulse at the two serially connected resistances. Simultaneously, voltage drop at one of the both resistors may be measured: either at the reference one, or at the unknown. Knowing the applied voltage and the voltage drop at one of the both resistances, may allow the value of the unknown resistance to be calculated. The described technique may demand minimum of additional components, which are needed to implement the fast-transient technique in an existing, in particular, digital potentiostat.

Specifically, determining of the membrane property, in particular a membrane resistance, may comprise generating the at least one fast-transient voltage signal $U_{gen,pulse}$ and applying it to a membrane comprising circuit serially connected with a reference resistor $R_{ref}$, wherein the membrane element has a resistance $R_{mem}$, recording a voltage $U_{meas,pulse}$ either at the reference resistor $R_{ref}$ or at the membrane element comprising circuit $R_{mem}$, determining the at least one membrane property by calculating the $R_{mem}$ from $U_{gen,pulse}$, $U_{meas,pulse}$ and $R_{ref}$. A simplified circuit may comprise the analyte sensor, represented as a simple Randle's circuit, the reference resistor $R_{ref}$, a measurement resistor $R_{meas}$, a shunt capacitor $C_{shunt}$, the signal generator device, in particular a voltage source, and a voltmeter (V). The Randle's circuit may comprise the charge transfer resistance $R_{ct}$, which represents the diffusion limited analyte current, double layer capacitance $C_{dl}$ at the electrode surface and the membrane element resistance $R_{mem}$. The signal generator device may be configured for applying a DC base voltage $U_{gen,base}$ and fast-transient voltage $U_{gen,pulse}$. During the DC base voltage is applied, the current flows through all four resistors in the circuit. There is no current flow through the capacitors, as they are charged to the corresponding level. The $R_{ct}$ may be a few orders of magnitude larger, than $R_{mem}$, such that the voltage drop at the $R_{mem}$ can be neglected in the first approximation. The same may be valid for the $R_{ref}$, which is chosen to be roughly the same value as the $R_{mem}$. The value for $R_{meas}$ may be chosen at the way, to get substantial voltage drop at it, which is then measured, e.g using an additional voltmeter or electrometer and converted in the response signal, also denoted sensor current signal. Thus, the value of the $R_{meas}$ may be roughly of the same order of magnitude as the $R_{ct}$. Since the voltage drop at the $R_{meas}$ is substantial, it may be compensated by the voltage source, which is in a feedback with the current measuring unit based on the $R_{meas}$. The calculation of the $R_{mem}$ may be done as

$$R_{mem} = R_{ref} \frac{U_{meas,pulse}}{U_{gen,pulse} - U_{meas,pulse}}$$

In order to perform the determining of the membrane property with high accuracy, acquisition of the response signal, in principle, must happen immediately after the fast-transient voltage signal is applied, because of a profile of the fast-transient voltage signal. Once the fast-transient voltage signal is applied at the analyte sensor, the analyte sensors' capacitive parts, such as double layer capacitance, are starting to charge. At the very beginning, the capacitive parts can be considered as a short cut, and, thus, corresponding resistive parts are short cut and do not play any role in the voltage drop across the analyte sensor. As longer the potential pulse continues, as more the capacitive parts in the analyte sensor may get charged, which may result in an additional voltage drop over these capacitors and, thus, also over the resistive parts so that the measurement may get inaccurate. In order to avoid undesired voltage distribution, as described above, the applied fast transient voltage signal must be as short as possible. Theoretically, the fast-transient voltage signal may be infinitely short. In practice, modern electronics may be sufficiently fast to reach a desired voltage magnitude within few ns. Usually, a limiting factor may be an acquisition speed of measurement electronics of a measurement unit such as of an analog-to-digital-converter (ADC), which is limited. The measurement electronics such as the ADC may convert an input voltage in digital form and compare it internally with internally generated and digitalized voltages (Successive-Approximation ADC). This process is called conversion. A minimal duration of this process may be determined by resolution and clock of the ADC, and takes, typically, few μs or less. Prior to this conversion, the input voltage may be sampled within an ADC channel. This is typically done by charging a small internal capacitor. Therefor the ADC may have corresponding switches: during the sampling, an external voltage to be determined is connected to the internal capacitor of the ADC. Once the capacitor is fully charged, it has the same voltage at its terminals as the input voltage to be determined. After that, the switches disconnect the external voltage and connect the capacitor to the internal converting and comparing unit. A limiting factor during this sampling phase may be the time, which is needed to charge the internal capacitor. Sampling time can be configured programmatically, but may not be lower, as needed for the full capacitor charge, otherwise the voltage at the internal capacitor does not reach the input value and the measurement is then wrong. Thus, the acquisition of the voltage value at the measurement electronics' input may take few microseconds because of the sampling and the conversion. Thus, the recorded voltage drop at the analyte sensor comprises certain error. In principle, the sampling time may be reduced by introducing further components in the schematics, like voltage follower, but this is not an option for a low-cost electronics.

As outlined above, it is impossible to record the voltage immediately after the pulse application. The present invention, in particular, proposes recording of response signals, in particular the voltage drop, at least two times and extrapolation of the recorded values of the response signal towards the time point $t_0$ of the application of the fast-transient voltage signal. The measurement unit, in particular the ADC, may be configured for precisely providing time ticks of voltage acquisition. Still, these two voltage acquisitions may be performed within shortest possible time after the application of the fast-transient voltage, in particular in view of an exponential character of charging of the capacitive parts. Since properties of these capacitive parts may not be well known and/or may also not be stable over time, it may be not possible and/or reliable to perform an exponential fitting. Therefore, the voltage acquisitions may be performed so fast, that there is still sufficiently linear range of an exponential.

The term "fast-transient voltage signal", also denoted as fast-transient voltage, as used herein is a broad term and is to be given its ordinary and customary meaning to a person of ordinary skill in the art and is not to be limited to a special or customized meaning. The term specifically may refer, without limitation, to at least one arbitrary voltage change in between two electrodes. The arbitrary voltage change may have fast transient signal flanks, in particular two very steep edges. The fast-transient voltage signal may comprise a square wave form and/or a sine wave form. The fast-transient voltage signal may comprise a non-continuous signal such as a pulse. Specifically, the fast-transient voltage signal may comprise a fast transition square wave.

The term "pulse" as used herein is a broad term and is to be given its ordinary and customary meaning to a person of ordinary skill in the art and is not to be limited to a special or customized meaning. The term specifically may refer, without limitation, to a signal having a transient change in the amplitude of the signal from a first value, also denoted baseline value, to a second value, followed by a return to the baseline value or at least approximately to the baseline value. The second value may be a higher or lower value than the baseline value. A pulse duration may be ≤ 50 μs, preferably ≤ 20 μs, more preferably ≤ 10 μs. The duration of the single pulse must be sufficiently long to be able to record its propagation. The duration of the single pulse must be preferentially short, in order to not excite the system electrochemically. The fast-transient voltage signal may be applied during at least one test sequence, for example a time sequence. The fast-transient voltage signal may be applied repeatedly, in particular periodically. The time distance between the cycles must be sufficiently long in order to keep the system at its steady-state. The fast-transient voltage signal may comprise a repeatable cycle, wherein the repeatable cycle comprises at least one signal flank. The pulse may comprise two edges: the leading edge or front edge, which is the first edge of the pulse and the trailing edge or back edge, which is the second edge of the pulse.

The terms first and second "value" may refer to regions or points of the fast-transient voltage signal, in particular its amplitude. The first value may be the baseline value. The first value may be a local and/or overall minimum of the fast-transient voltage signal. The first value may be a first plateau of the fast-transient voltage signal. The first value may refer to a time point with no voltage is applied to the measurement electrodes. The first value may be the DC polarization voltage of the sensor. The second value may be a local and/or overall extremum of the fast-transient voltage signal. The second value may be a second plateau of the fast-transient voltage signal, which may be reached during application of the fast-transient voltage. The second value may be extremum of the fast-transient voltage signal.

The term "signal flank" as used herein is a broad term and is to be given its ordinary and customary meaning to a person of ordinary skill in the art and is not to be limited to a special or customized meaning. The term specifically may refer, without limitation, to transition of a signal amplitude from low to high signal value or from high to low signal value. The signal flank may be a rising signal flank or a falling signal flank. The signal flank of the fast-transient voltage signal may have a change in signal from the first value of the signal flank to the second value of the signal flank in a microsecond to nanosecond range. The signal flank of the fast-transient voltage signal may have a change in signal from the second value of the signal flank to the first value of the signal flank in a microsecond to nanosecond range. The signal flank may also be referred to as edge.

The fast-transient voltage signal may have a low-to-high transition of a signal amplitude, which is equivalent to rising or positive signal flank, or high-to-low transition of a signal amplitude, which is equivalent to falling or negative signal flank. The fast-transient voltage signal may have steep edges. The signal flank, in particular edge, of the fast-transient voltage signal may have a change from the first value to the second value in a microsecond to nanosecond range. The signal flank of the fast-transient voltage signal may have a change from the second value to the first value in a microsecond to nanosecond range. Specifically, the fast transition square wave may have a change in voltage from the first value to the second value below 50 ns, preferably below 20 ns. The change in voltage from the first value to the second value may be even faster and may be only limited by electronics such as by a fast-transient voltage generator, e.g. comprising at least one digital to analog converter (DAC) and/or at least one digital output (DO) or the like, or the measurement unit, e.g. comprising at least one voltage amplifier, ADC, or the like. The faster the change of voltage (higher slew rate) and the sharper a transition to a plateau, the more precise the membrane property can be determined.

The term "fast-transient" as used herein is a broad term and is to be given its ordinary and customary meaning to a person of ordinary skill in the art and is not to be limited to a special or customized meaning. The term specifically may refer, without limitation, to a time range between first and second values of the signal flank. The fast-transient voltage signal may have a rising signal flank and a falling signal flank. The fast-transient voltage signal may have steep edges. Specifically, the fast transition square wave may have a change in signal from the first value of the signal flank to the second value of the signal flank below 50 ns, preferably below 20 ns. The change in signal from the first value of the signal flank to the second value of the signal flank may be even faster and may be only limited by electronics such as by an analog-to-digital-converter. The faster the flank and the sharper the transition to the plateau, the more resolution may be between the ohmic part of the system resistance and the capacitive part of the system capacitance.

The duration of the single fast-transient voltage signal must be sufficiently long to record the response voltage. The duration of the single fast-transient voltage signal must be sufficiently short, in order to avoid system perturbation.

Without wishing to being bound by theory, the fast-transient voltage signal, in particular the voltage pulse, is so short, in particular ultrashort, that no faradaic currents are generated and that an electrochemical system of the analyte sensor is not disturbed and brought out of equilibrium. The ultrashort voltage of the fast-transient voltage signal for determining the membrane property may allow that a measurement signal for determining the analyte concentration can be undisturbed determined. The ultrashort voltage signal may prevent that side reaction occur. Moreover, the method according to the present invention may allow to stay in the so-called time domain such that there is no need to transform to the so-called frequency domain.

An amplitude of the fast-transient voltage may vary in a broad range and must be optimized for a given set-up. Generally, the lower limit may be limited by the readout technique, which must record the response voltage, mostly by its input range and resolution and may require an additional sufficiently fast voltage amplifier.

The fast-transient voltage signal may comprise a repeatable cycle, wherein the repeatable cycle comprises at least one signal edge. The fast-transient voltage signal may be applied during at least one test sequence, for example a time sequence. The fast-transient voltage signal may be applied repeatedly, in particular periodically. The interval between the cycles may be sufficiently long in order to let the double layer capacitance and the shunt capacitor to recharge to their previous steady-state voltage. The discharge of these capacitances after stop of the fast-transient voltage signal applying, as described above, means current flow opposite to the analyte current and thus distortion of the signal. Thus, the data acquisition for the recharging time may be stopped or the corresponding acquired samples may be ignored.

The fast-transient voltage signal may be applied repeatedly to the measurement electrodes, in particular in time intervals from minutes to seconds. For example, the fast-transient voltage signal may be applied repeatedly in 5 minutes-intervals.

The fast-transient voltage signal may be generated by at least one signal generator device. The term "signal generator device" generally refers to a device, for example a voltage source, being configured to generate a voltage signal. The "signal generator device" may also be referred to as "voltage generating device". The signal generator device may comprise at least one voltage source. The signal generator device may comprise at least one function generator selected from the group consisting of: at least one square wave generator and at least one sine wave generator. The signal generator device may also generate a single pulse which may be unsymmetrical. "Unsymmetrical" in this context means that a first pulse may be different from a second pulse and/or a third pulse and/or any other subsequent pulse. The signal generator device may be part of measurement electronics of the analyte sensor and/or may be connected to the analyte sensor and may be designed as a separate device. The signal generator device may be configured for applying the fast-transient voltage signal to the measurement electrodes. The fast-transient voltage signal may be applied to at least two measurement electrodes in at least one signal application step.

The term "applying the fast-transient voltage signal to the measurement electrodes" as used herein is a broad term and is to be given its ordinary and customary meaning to a person of ordinary skill in the art and is not to be limited to a special or customized meaning. The term specifically may refer, without limitation, to applying the fast-transient voltage signal to one of the measurement electrodes, in particular to the working electrode. The fast-transient voltage signal to the measurement electrodes is applied at an application time to. The term "application time" as used herein is a broad term and is to be given its ordinary and customary meaning to a person of ordinary skill in the art and is not to be limited to a special or customized meaning. The term specifically may refer, without limitation, to the time point at which the fast-transient voltage signal is applied to the measurement electrodes. The application time may be defined and/or pre-defined by the signal generator. The signal generator and/or at least one data storage device may be configured for storing the application time.

The term "response signal" as used herein is a broad term and is to be given its ordinary and customary meaning to a person of ordinary skill in the art and is not to be limited to a special or customized meaning. The term specifically may refer, without limitation, to measured propagation of the applied fast-transient voltage signal. The terms "response signal" and "propagation" are used herein as synonyms. The response signal may be a change of the applied fast-transient voltage signal. The response signal may directly or indirectly refer to equivalent series resistance of the analyte sensor. The response signal may be the ohmic and capacitive characterization of the analyte sensor in its in-vivo surroundings. In particular, the response signal does not relate to current response. The response voltage may be determined either at a reference resistor or at the membrane element.

The method may comprise measuring at least two response signals, i.e. the first response signal $U_1$ and the second response signal $U_2$. The terms "first" and "second" are solely used in order to enable differentiation between two terms and, in the case of the term "response signal". Thus, the method may comprise measuring further response signals, e.g. before and/or after and/or between the first and second response signal. However, the first response signal $U_1$ is measured at a first time $t_1$ and the second response signal $U_2$ is measured at a second time $t_2$ with to $\neq t_1 \neq t_2$, wherein the application time to precedes the first time $t_1$ and the second time $t_2$. The first time and the second time may be arbitrary time points which fulfill the mentioned requirements. The first time $t_1$ may be in a first time range after the application time $t_0$. The second time $t_2$ may be in a second time range after the first time $t_1$. Lower limits of the first time range and the second time range may be defined by time resolution of at least one measurement unit configured for receiving the first response signal and the second response signal. Upper limits of the first time range and the second time range may be defined by charging characteristics of the capacitive parts of the analyte sensor. The voltage pulse may induce flow of capacitive and faraday currents. In order to maintain the sensor integrity, the faraday current flow should be excluded. Therefore, the voltage pulse amplitude and duration must be fitted to the sensor capacitance and the membrane resistance and be as low and short respectively, as possible to avoid inducing the faraday current flow. Considering an analyte sensor with a capacitance <10 nF and $R_{mem}$ < 10 kOhm and the pulse amplitude of 1.5 V, the faraday current starts to flow after roughly 3 $\mu$s. Thus this duration shall not be exceeded in order to avoid faraday currents. However, the pulse duration may be longer if sensor capacitance is larger and/or $R_{mem}$ is higher. Furthermore, the faraday current may be allowed, once the sensor design considers it. The term "capacitive parts" as used herein is a broad term and is to be given its ordinary and customary meaning to a person of ordinary skill in the art and is not to be limited to a special or customized meaning. The term specifically may refer, without limitation, to any elements of the analyte sensor configured for storing electrical energy, such as the double layer capacitances. The term "charging characteristics" as used herein is a broad term and is to be given its ordinary and customary meaning to a person of ordinary skill in the art and is not to be limited to a special or customized meaning. The term specifically may refer, without limitation, to charging behavior as a function of time and/or time dependence of charging. The charging characteristics may follow a charging curve Q(t). The charging curve may be an exponential curve. Thus, the measuring of the first response signal and the second response signal may be performed in view of the exponential character of charging of the capacitive parts. The measuring of the first response signal and the second response signal may be performed within shortest possible time after the application of the fast-transient voltage. Since properties of the capacitive parts may not be well known and/or may also not be stable over time, it may be not possible and/or reliable to perform an exponential fitting. Therefore, the voltage acquisitions may be performed so fast, that the charging curve is still in its linear part. The first time $t_1$ may be in the range from 1 $\mu$s to 5 $\mu$s after the application time $t_0$. The second time $t_2$ may be in the range from 1 $\mu$s to 5 $\mu$s after the first time $t_1$. The measurement unit, in particular the ADC, may be configured for determining the first and second time, in particular with high precision.

The measuring of the first response signal and the second response signal may be performed using the at least one measurement unit. The term "measurement unit" as used herein is a broad term and is to be given its ordinary and customary meaning to a person of ordinary skill in the art and is not to be limited to a special or customized meaning. The term specifically may refer, without limitation, to an arbitrary device, preferably at least one electronic device, which may be configured to detect at least one signal, in particular the response signal. The measurement unit may be configured for measuring the first and second response signals generated in response to fast-transient voltage signal. The measurement unit may further be configured for measuring the current at the counter electrode for determining a concentration of at least one analyte in bodily fluid. The measurement unit may be configured for receiving the response signal and the current at the counter electrode at the same time or at at least two different time points.

The measurement unit may comprise at least one potentiostat such as at least one digital potentiostat or at least one analog potentiostat. The analyte sensor may comprise and/or may be connected to the measurement unit, in particular to the at least one potentiostat or galvanostat. The measurement unit may be configured for determining the concentration of the analyte. Operating principles of potentiostats and galvanostats are generally known to the person skilled in the art. In the following the measurement unit will be described with reference to a potentiostat.

The potentiostat may be configured for generating and/or applying of at least one measurement voltage signal, in particular a polarizing potential or voltage. As used herein, the term "measurement voltage signal" may refer to a voltage signal used for determining the concentration of the analyte. The measurement voltage signal may be different to the fast-transient voltage signal. In particular, the measurement voltage signal may be longer compared to the fast-transient voltage signal. The measurement voltage signal may be a permanent signal, not a pulsed one. The measurement voltage signal may be adjusted from time to time or continuously in order to give the analyte sensor its polarization voltage, preferably, in order to keep the predefined polarization voltage at the analyte sensor. The measurement voltage signal may be a continuous direct current (DC) signal which polarizes the electrochemical cell, and serves as the "motor" for the amperometric measurement of the analyte reducing or oxidizing GOx across the electrochemical cell. The fast-transient voltage signal may be a voltage pulse with high frequency that only characterizes the capacitive and ohmic parts of the electrochemical cell. Therefore, the measurement voltage signal and the fast-transient voltage signal may not influence each other, since they have completely different time domains.

In a two-electrode system, the measurement voltage signal and the fast-transient voltage signal may be applied to the same electrodes. In a three-electrode system a working voltage is determined and controlled between the working electrode and the reference electrode. In order to achieve this, the potentiostat may regulate the potential of the counter electrode. The fast-transient voltage signal may be applied between the counter and the working electrode or between the working and the reference electrode or between the counter and the reference electrode.

The potentiostat may be configured for monitoring and maintaining the potential between the reference electrode and the working electrode. The potentiostat may be configured for monitoring and maintaining the potential between the combined counter-reference electrode and the working electrode. The potentiostat may be configured for maintaining the desired polarization voltage, for example 50 mV, between the reference electrode and the working electrode or between the working electrode and the combined counter-reference electrode. The current flowing between the working and the counter or the combined counter-reference electrode may be measured at the working or the counter or the combined counter-reference electrode. The reference electrode may be used to monitor the potential of the working electrode.

The measuring of the first response signal and the second response signal may be performed using the at least one reference resistor. Before the application of the fast-transient voltage signal the measurement unit, in particular the potentiostat, may measure the measurement voltage only. During the application of the fast-transient voltage signal, the potentiostat determines the sum of the measurement voltage signal and the fast-transient voltage signal. The potentiostat may be configured for determining the propagation of the fast-transient voltage signal applied to the working electrode. The potentiostat may be configured for determining a change or difference $\Delta V_{ex}$ of the voltage signal at the reference resistor before application of the fast-transient voltage signal and during the application of the fast-transient voltage signal. The potentiostat may be configured for determining a change or difference $\Delta V_{prop}$ of voltage at the working electrode before application of the fast-transient voltage signal and during the application of the fast-transient voltage signal.

The reference resistor may have a resistance, also denoted reference resistance, suitable for determining a value to be measured such as the electrical resistance of the membrane element. The reference resistance may be an average value determined, specifically pre-determined, from a plurality of reference measurements. The reference resistance may reflect the measurement range of the membrane element. The reference resistance may reflect required measurement tolerances which have to be maintained for correct membrane element property, in particular membrane resistance.

An equivalent circuit of the electrochemical system of the analyte sensor, may comprise for each of the working electrode and the counter electrode a double layer capacitance in parallel with a charge transfer resistance, as outlined above. The resistance of the electrolyte between the working electrode and the reference electrode may be given by an electric resistance $R_2$ and the resistance of the electrolyte between the counter electrode and the reference electrode may be given by an electric resistance $R_1$. The resistance $R_2$ may further be dependent on properties of the membrane element.

For measuring the response signals, additional components may be used, in particular, in addition to the components of the potentiostat as described above. For example, the measurement unit may comprise additional capacitors and/or additional resistors. Specifically, the fast-transient voltage signal may be applied to one of the measurement electrodes, in particular the working electrode, in series with the reference resistance, denoted $R_3$ or $R_{ref}$. $R_{ref}$ may be a known reference resistance such as a predetermined reference resistance. As outlined above, the reference resistance may reflect the measurement range of the cell. The reference resistance may reflect required measurement tolerances which must be maintained for correct system resistances. The reference resistance may be selected suitable for determining a value to be measured such as the electrical resistance of the membrane element. The fast-transient voltage signal may be determined by using the reference resistor. Before the application of the fast-transient voltage signal the potentiostat determines the measurement voltage signal only. After the application of the fast-transient voltage signal the potentiostat determines the sum of the measurement voltage signal and the fast-transient voltage signal.

Step c) comprises determining the response signal $U_0$ at the application time $t_0$ by evaluating the first response signal $U_1$ and the second response signal $U_2$. The term "evaluating" as used herein is a broad term and is to be given its ordinary and customary meaning to a person of ordinary skill in the art and is not to be limited to a special or customized meaning. The term specifically may refer, without limitation, to a process of extrapolating and/or deriving the response signal $U_0$ from the measurements of the first response signal $U_1$ and the second response signal $U_2$. Thus, the response signal $U_0$ may not be measured directly but may be evaluated from the first response signal $U_1$ and the second response signal $U_2$. The evaluating may comprise applying at least one fit procedure. The fit procedure may comprise fitting the first response signal $U_1$ and the second response signal $U_2$ by using at least one fit function, in particular a linear fit function $U(t) = b \cdot t + a$ with b being the slope and a the intercept. By using the measured points $(t_1, U_1)$ and $(t_2, U_2)$ the fit parameters b and a may be determined. The determined linear function may be used for extrapolation of the measured first response signal at $t_1$ and the second response signal at $t_2$ towards the time point to of the application of the fast-transient voltage signal for determining $U_0$.

Step d) comprises determining the at least one membrane property by evaluating of the response signal $U_0$ at the application time $t_0$. In particular, the evaluating of the response signal $U_0$ comprises determining equivalent series resistance of the analyte sensor and determining the at least one membrane property from the equivalent series resistance of the analyte sensor. The evaluating of the response signal $U_0$ at the application time to may comprise determining equivalent series resistance of the electrochemical system and determining the at least one membrane property from the equivalent series resistance of the electrochemical system. In order to measure the membrane property, in particular equivalent series resistance of the electrochemical system, the fast-transient voltage signal may be sent to the working electrode. The edges of the fast-transient voltage signal are very steep such that the additional capacitors and equivalent capacitors of the electrochemical system of the analyte sensor act like short-circuits. The equivalent series resistance of the electrochemical system may be determined by

$$R_1 + R_2 = R_3 \frac{\Delta V_{prop}}{\Delta V_{ex} - \Delta V_{prop}} =$$

$$R_3 \frac{V_{prop,duringPulse} - V_{prop,beforePulse}}{\left(V_{ex,duringPulse} - V_{ex,beforePulse}\right) - \left(V_{prop,duringPulse} - V_{prop,beforePulse}\right)}$$

wherein $v_{prop,beforePulse}$ refers to the voltage at the working electrode before applying the fast-transient voltage signal, $V_{prop,duringPulse}$ refers to the voltage at the working electrode during applying the fast-transient voltage signal, $V_{ex,beforePulse}$ refers to the voltage signal at the reference resistor before applying the fast-transient voltage signal,

$V_{ex,duringPulse}$ refers to the voltage signal at the reference resistor during applying the fast-transient voltage signal. Before the application of the fast-transient voltage signal $V_{ex,beforePulse}$ may refer to a voltage at the reference resistor in response to the measurement voltage signal. After the application of the fast-transient voltage signal $V_{ex,duringPulse}$ may refer to the voltage at the reference resistor in response to the measurement voltage signal and due to the propagation of the fast-transient voltage signal.

The technical realization of the measurement setup may be simple and requires only a minimum number of additional components in addition to the known potentiostat. The determined response signals may not require further processing and may be directly digitalized.

The measured response signals may provide absolute values and not relative changes. The determined electrical resistance may be very selective to the membrane property. In particular, the measured electrical resistance may not comprise resistance relating to charge transfer processes of the electrochemical system. Thus, it may be possible to exclude the influences, e.g. of the test chemistry, to the response signals.

As outlined above, the analyte sensor may be an in vivo sensor, specifically an in vivo continuous glucose sensor. The method may be an in-process control. The method may be performed during in-vivo measurement. The method may be performed in-operando. Specifically, the method may be performed during determining of the concentration of the analyte. Additionally or alternatively, the method may be performed during manufacturing of the analyte sensor. For example, the manufacturing process may comprise at least one calibration, wherein the analyte sensor may be operated with a sample of known analyte concentration. The method may be used for providing a factory calibrated analyte sensor. Not each sensor of the given batch may be calibrated, but some of the analyte sensors.

The method may comprise at least one failsafe step. As used herein, the term "failsafe step" refers to at least one step ensuring to prevent generating and/or determining and/or displaying unreliable or false measurement values. The failsafe step may be triggered depending on the determined membrane property. The failsafe step may comprise generating at least one information about a condition of the membrane element. The term "condition of the membrane element" as used herein is a broad term and is to be given its ordinary and customary meaning to a person of ordinary skill in the art and is not to be limited to a special or customized meaning. The term specifically may refer, without limitation, to information about suitability of the membrane element to be used in the analyte sensor for determining the concentration of the analyte. For example, the information about the condition may comprise information about aging and/or mechanical stability. The condition of the membrane element may comprise information about manufacturing tolerances of the membrane thicknesses through dispensing, screen printing or other which lead to these differences in diffusion. The method according to the present invention may allow to identify differences in lot runs from material suppliers, or changes when a supplier changes something in the makeup of the membrane material. The failsafe step may further comprise detecting excessive moisture across the counter electrode and the working electrode. The failsafe step may comprise comparing the determined membrane property with at least one pre-determined or pre-defined reference value. The failsafe step may comprise storing, e.g. within a measurement engine electronic, for example, of the evaluation device, the pre-determined and/or pre-defined reference value, in particular a resistance limit. For example, the determined membrane property deviates from the pre-determined or pre-defined reference value. For example, an expected membrane element resistance may be 2 kΩ. If the determined membrane element resistance is very different from what is expected, the analyte sensor may be considered as failed sensor. Having something very different over long time may indicate a failed sensor. Having the determined membrane element resistance value close or equal zero may indicate shortcut, having the determined membrane element resistance out of range may indicate circuit brake. For example, in case the determined membrane property deviates from the pre-determined or pre-defined reference value, the determining of the concentration of the analyte may be stopped and/or determined concentration values may be rejected and/or the analyte sensor may be rejected for use or further use. The failsafe step may be performed before and/or during determination of the at least one analyte in bodily fluid. The failsafe step may be performed repeatedly, for example in a pre-defined interval, such as every minute or every 5 minutes.

However, other embodiments and time intervals are possible. Based on the comparison, in the failsafe step, at least one failsafe decision may be determined and/or at least one failsafe action may be performed. For example, the failsafe step may comprise issuing and/or displaying an error message in case the information on the electrical resistance of the membrane element exceeds the resistance limit. For example, the failsafe step may comprise preventing issuing and/or displaying the analytical result in case the electrical resistance of the membrane element exceeds the resistance limit. The failsafe step may comprise issuing and/or displaying an error message in case the electrical resistance of the membrane element exceeds the resistance limit. The failsafe step may comprise displaying a warning message in case the electrical resistance of the membrane element exceeds the resistance limit. The failsafe step may comprise request to remove the analyte sensor in case the electrical resistance of the membrane element exceeds the resistance limit.

In a further aspect, a method for determining a concentration of at least one analyte in bodily fluid using at least one analyte sensor is disclosed. The analyte sensor comprises at least two measurement electrodes. At least one of the measurement electrodes comprises at least one membrane element having at least one membrane property. The method comprises determining the at least one membrane property of the analyte sensor according to the present invention and according to one or more of the embodiments of the method as disclosed above or as disclosed in further detail below. The method comprises at least one analyte measurement step. In the measurement step at least one measurement value of the concentration of the analyte is determined.

One or more of the method steps may be performed in parallel and/or in a time overlapping fashion. Further, one or more of the method steps may be performed repeatedly. Further, additional method steps may be present which are not listed. For definitions of the features of the method and for optional details of the method for determining the concentration of the analyte, reference may be made to one or more of the embodiments of the method for determining the membrane property as disclosed above or as disclosed in further detail below.

The term "determining a concentration of at least one analyte" generally refers to a quantitative detection of the at least one analyte. As a result of the determination, at least one signal, such as at least one measurement signal, and/or at least one measurement value may be produced and/or provided which characterizes an outcome of the determination. The signal specifically may be or may comprise at least one electronic signal such as at least one voltage and/or at least one current. The at least one signal may be or may comprise at least one analogue signal and/or may be or may comprise at least one digital signal.

As outlined above, the method comprises at least one analyte measurement step. In the analyte measurement step the measurement voltage signal may be applied to the working electrode such that a constant potential may be applied between the working electrode and the reference electrode such that a current produced at the working electrode flows towards the counter electrode. The current may be measured at the counter electrode using I/U converter and an analog to digital converter (ADC) channel. The method furthermore may comprise at least one evaluation step, wherein current is evaluated. At least one evaluation device may be used for evaluating the measured current and for determining the concentration of the analyte therefrom. As used herein, the term "evaluation device" generally refers to an arbitrary device being configured to derive at least one item of information from data. The evaluation device may be configured to derive the at least one item of information regarding the presence and/or concentration of the analyte in the bodily fluid from the current. As an example, the evaluation device may be or may comprise one or more integrated circuits, such as one or more application-specific integrated circuits (ASICs), and/or one or more data processing devices, such as one or more computers, preferably one or more microcomputers and/or microcontrollers. Additional components may be comprised, such as one or more preprocessing devices and/or data acquisition devices, such as one or more devices for receiving and/or preprocessing of the electrode signals, such as one or more converters and/or one or more filters. Further, the evaluation device may comprise one or more data storage devices. Further, as outlined above, the evaluation device may comprise one or more interfaces, such as one or more wireless interfaces and/or one or more wire-bound interfaces. The evaluation device may comprise a microprocessor, a cellular phone, a smart phone, a personal digital assistant, a personal computer, or a computer server.

The invention further discloses and proposes a computer program including computer-executable instructions for performing the method for determining a concentration of at least one analyte and/or the method for determining at least one membrane property according to the present invention in one or more of the embodiments enclosed herein, when the program is executed on a computer or computer network. Specifically, the computer program may be stored on a computer-readable data carrier. Thus, specifically, one, more than one or even all of method steps, as indicated above, may be performed by using a computer or a computer network, preferably by using a computer program.

The invention further discloses and proposes a computer program product having program code means, in order to perform the method for determining a concentration of at least one analyte and/or the method for determining at least one membrane property according to the present invention in one or more of the embodiments enclosed herein, when the program is executed on a computer or computer network. Specifically, the program code means may be stored on a computer-readable data carrier.

Further, the invention discloses and proposes a data carrier having a data structure stored thereon, which, after loading into a computer or computer network, such as into a working memory or main memory of the computer or computer network, may execute the methods according to one or more of the embodiments disclosed herein.

The invention further proposes and discloses a computer program product with program code means stored on a machine-readable carrier, in order to perform at least one of the methods according to one or more of the embodiments disclosed herein, when the program is executed on a computer or computer network. As used herein, a computer program product refers to the program as a tradable product. The product may generally exist in an arbitrary format, such as in a paper format, or on a computer-readable data carrier. Specifically, the computer program product may be distributed over a data network.

Finally, the invention proposes and discloses a modulated data signal which contains instructions readable by a computer system or computer network, for performing the methods according to one or more of the embodiments disclosed herein.

Preferably, referring to the computer-implemented aspects of the invention, one or more of the method steps or even all of the method steps of at least one of the methods according to one or more of the embodiments disclosed herein may be performed by using a computer or computer network. Thus, generally, any of the method steps including provision and/or manipulation of data may be performed by using a computer or computer network. Generally, these method steps may include any of the method steps, typically except for method steps requiring manual work, such as providing the samples and/or certain aspects of performing the actual measurements.

Specifically, the present invention further discloses:

- A computer or computer network comprising at least one processor, wherein the processor is adapted to perform at least one of the methods according to one of the embodiments described in this description,
- a computer loadable data structure that is adapted to perform at least one of the methods according to one of the embodiments described in this description while the data structure is being executed on a computer,
- a computer program, wherein the computer program is adapted to perform at least one of the methods according to one of the embodiments described in this description while the program is being executed on a computer,
- a computer program comprising program means for performing at least one of the methods according to one of the embodiments described in this description while the computer program is being executed on a computer or on a computer network,
- a computer program comprising program means according to the preceding embodiment, wherein the program means are stored on a storage medium readable to a computer,
- a storage medium, wherein a data structure is stored on the storage medium and wherein the data structure is adapted to perform at least one of the methods according to one of the embodiments described in this description after having been loaded into a main and/or working storage of a computer or of a computer network, and
- a computer program product having program code means, wherein the program code means can be stored or are stored on a storage medium, for performing at least one of the methods according to one of the embodiments described in this description, if the program code means are executed on a computer or on a computer network.

In a further aspect of the present invention, an analytical system for determining a concentration of at least one analyte in bodily fluid is disclosed. The analytical system comprises at least one analyte sensor, wherein the analyte sensor comprises at least two measurement electrodes, wherein at least one of the measurement electrodes comprises at least one membrane element having at least one membrane property. The analytical system comprises at least one signal generator device configured for generating at least one fast-transient voltage signal, wherein the signal generator device is configured for applying the fast-transient voltage signal to the two measurement electrodes. The analytical system comprises at least one measurement unit configured for measuring a first response signal $U_1$ at a first time $t_1$ and a second response signal $U_2$ at a second time $t_2$ with $t_0 \neq t_1 \neq t_2$. The application time $t_0$ precedes the first time $t_1$ and the second time $t_2$. The analytical system comprises at least one evaluation device, wherein the evaluation device is configured for determining a response signal $U_0$ at the application time $t_0$ by evaluating the first response signal $U_1$ and the second response signal $U_2$. The evaluation device is configured for determining the at least one membrane property by evaluating of the response signal $U_0$ at the application time $t_0$.

The analytical system may be configured for performing the methods according to the present invention. For definitions of the features of the analytical system and for optional details of the analytical system, reference may be made to one or more of the embodiments of the methods as disclosed above or as disclosed in further detail below.

As further used herein, the term "system" refers to an arbitrary set of interacting or interdependent component parts forming a whole. Specifically, the components may interact with each other in order to fulfill at least one common function. The at least two components may be handled independently or may be coupled or connectable. Thus, the term "analytical system" generally refers to a group of at least two elements or components which are capable of interacting in order to perform at least one analytical detection, specifically at least one analytical detection of at least one analyte of the sample. The analytical system may be an apparatus, specifically comprising at least two components.

The analyte sensor may be a two-electrodes sensor or a three-electrodes sensor. The analyte sensor may comprise two measurement electrodes or three measurement electrodes. The measurement electrodes may be arranged on opposing sides of the analyte sensor.

Summarizing the findings of the present invention, the following embodiments are preferred:

Embodiment 1. A method for determining at least one membrane property of an analyte sensor, wherein the analyte sensor comprises at least two measurement electrodes, wherein at least one of the measurement electrodes comprises at least one membrane element having at least one membrane property, the method comprising the following steps:

a) generating at least one fast-transient voltage signal and applying the fast-transient voltage signal to the measurement electrodes at an application time to;
b) measuring a first response signal $U_1$ at a first time $t_1$ and a second response signal $U_2$ at a second time $t_2$ with $t_0 \neq t_1 \neq t_2$, wherein the application time $t_0$ precedes the first time $t_1$ and the second time $t_2$;

c) determining a response signal $U_0$ at the application time $t_0$ by evaluating the first response signal $U_1$ and the second response signal $U_2$;

d) determining the at least one membrane property by evaluating of the response signal $U_0$ at the application time $t_0$.

Embodiment 2. The method according to embodiment 1, wherein the evaluating of the response signal $U_0$ in step d) comprises determining equivalent series resistance of the analyte sensor and determining the at least one membrane property from the equivalent series resistance of the analyte sensor.

Embodiment 3. The method according to any one of embodiments 1 or 2, wherein the first time $t_1$ is in the range from 1 $\mu$s to 5 $\mu$s after the application time to.

Embodiment 4. The method according to any one of embodiments 1 to 3, wherein the second time $t_2$ is in the range from 1 $\mu$s to 5 $\mu$s after the first time $t_1$.

Embodiment 5. The method according to any one of embodiments 1 to 4, wherein the analyte sensor is an in vivo sensor.

Embodiment 6. The method according to any one of embodiments 1 to 5, wherein the method is performed during in vivo measurement.

Embodiment 7. The method according to any one of embodiments 1 to 6, wherein the method is performed during manufacturing of the analyte sensor.

Embodiment 8. The method according to any one of embodiments 1 to 7, wherein the method comprises at least one failsafe step, wherein the failsafe step is triggered depending on the determined membrane property.

Embodiment 9. The method according to any one of embodiments 1 to 8, wherein the membrane property is permeability of the membrane element.

Embodiment 10. The method according to any one of embodiments 1 to 9, wherein the fast-transient voltage signal has a square wave form or a sine wave signal form.

Embodiment 11. The method according to any one of embodiments 1 to 10, wherein the fast-transient voltage signal comprises a non-continuous signal such as a pulse, wherein a pulse duration is $\leq$ 20 $\mu$s, preferably $\leq$ 10 $\mu$s.

Embodiment 12. Method for determining a concentration of at least one analyte in bodily fluid using at least one analyte sensor, wherein the analyte sensor comprises at least two measurement electrodes, wherein at least one of the measurement electrodes comprises at least one membrane element having at least one membrane property, wherein the method comprises determining at least one membrane property of the analyte sensor according to any one of embodiments 1 to 11, wherein the method comprises at least one analyte measurement step, wherein in the measurement step the concentration of the analyte is determined.

Embodiment 13. A computer program comprising program means for performing the method according to any one of embodiments 1 to 11 and/or the method according to embodiment 12 while the computer program is being executed on a computer or on a computer network.

Embodiment 14. An analytical system for determining a concentration of at least one analyte in bodily fluid, wherein the analytical system comprises at least one analyte sensor, wherein the analyte sensor comprises at least two measurement electrodes, wherein at least one of the measurement electrodes comprises at least one membrane element having at least one membrane property, wherein the analytical system comprises at least one signal generator device configured for generating at least one fast-transient voltage signal, wherein the signal generator device is configured for applying the fast-transient voltage signal to the two measurement electrodes, wherein the analytical system comprises at least one measurement unit configured for measuring a first response signal $U_1$ at a first time $t_1$ and a second response signal $U_2$ at a second time $t_2$ with $t_0 \neq t_1 \neq t_2$, wherein the application time to precedes the first time $t_1$ and the second time $t_2$, wherein the analytical system comprises at least one evaluation device, wherein the evaluation device is configured for determining a response signal $U_0$ at the application time $t_0$ by evaluating the first response signal $U_1$ and the second response signal $U_2$, wherein the evaluation device is configured for determining the at least one membrane property by evaluating of the response signal $U_0$ at the application time to.

Embodiment 15. The analytical system according to embodiment 14, wherein the analyte sensor comprises two measurement electrodes or three measurement electrodes.

Embodiment 16. The analytical system according to any one of embodiments 14 or 15, wherein the measurement electrodes are arranged on opposing sides of the analyte sensor.

Embodiment 17. The analytical system according to any one of embodiments 14 to 16, wherein the analytical system is configured for performing the method according to any one of embodiments 1 to 11 and/or the method according to embodiment 12.

Short description of the Figures

Further optional features and embodiments will be disclosed in more detail in the subsequent description of embodiments, preferably in conjunction with the dependent claims. Therein, the respective optional features may be realized in an isolated fashion as well as in any arbitrary feasible combination, as the skilled person will realize. The scope of the invention is not restricted by the preferred embodiments. The embodiments are schematically depicted in the Figures. Therein, identical reference numbers in these Figures refer to identical or functionally comparable elements.
In the Figures:

Figure 1     shows a schematic representing at least one analytical system according to the present invention;

Figure 2     shows a flowchart of a method for determining at least one membrane property of an analyte sensor according to the present invention; and

Figure 3     shows exemplary development of measured voltage as a function of time.

Detailed description of the embodiments

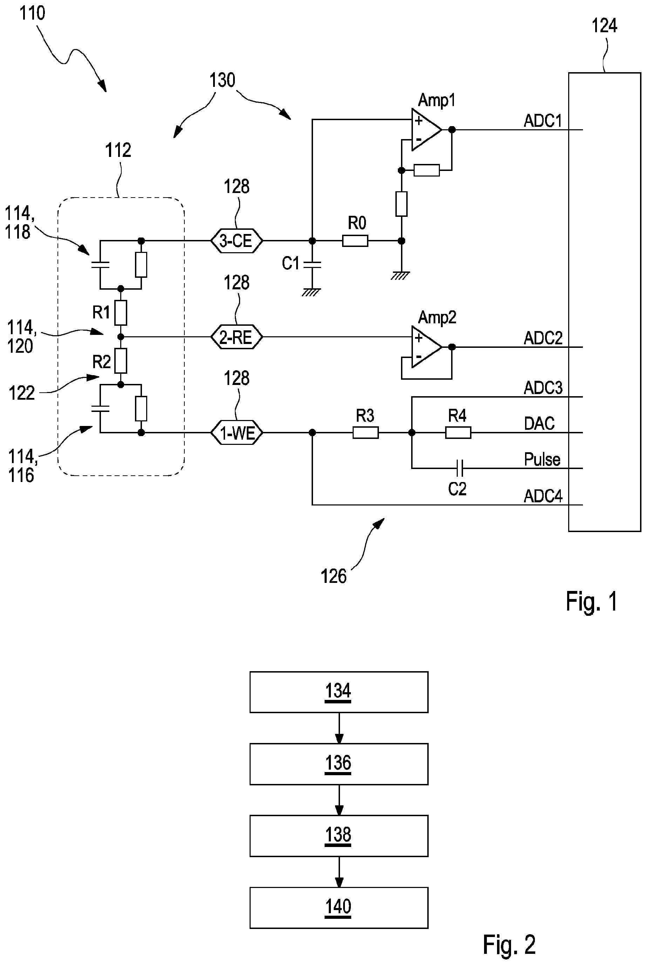

Figure 1 shows a schematic representing at least one analytical system 110 for determining a concentration of at least one analyte in bodily fluid according to the present invention. The analytical system 110 comprises at least one analyte sensor 112 shown here as equivalent circuit.
The analyte may be or may comprise an arbitrary chemical substance or chemical compound which may take part in the metabolism of the user, such as at least one metabolite. As an example, the at least one analyte may be selected from the group consisting of glucose, cholesterol, triglycerides, lactate. Additionally or alternatively, however, other types of analytes may be determined and/or any combination of analytes may be determined.
In an embodiment, the analyte sensor 112 may be an optical sensor.
The analyte sensor 112 may be an in vivo sensor. The analyte sensor 112 may be configured for being at least partially implanted into a body tissue of a user. The analyte sensor 112 may a subcutaneous analyte sensor. The analyte sensor 112 may be configured for implantation into a body tissue of the user. More specifically the analyte sensor 112 may be configured for continuous monitoring of the analyte.
The analyte sensor 112 comprises at least two measurement electrodes 114. The at least two measurement electrodes 114 may be designed such that an electrochemical reaction may take place at one or more of the electrodes. Thus, the measurement electrodes 114 may be embodied such that an oxidation reaction and/or reduction reaction may take place at one or more of the electrodes.
One of the measurement electrodes 114 may be designed as working electrode 116. In Figure 1 for the working electrode 116 a capacitance representing the electric double layer and a resistance representing the charge transfer resistance is shown. The working electrode 116 may comprise at least one test chemical. The working electrode 116 may fully or partially be covered with at least one test chemical, specifically at least one test chemical comprising at least one enzyme for detecting the at least one analyte. As an example, glucose oxidase (GOx) or glucose dehydrogenase (GDH) may be used. The test chemical, further, may comprise additional materials, such as binder materials, electrode particles, mediators or the like. Thus, as an example, the test chemical may comprise at least one enzyme, carbon particles, a polymer binder and $MnO_2$-particles. In another preferred embodiment, the test chemical may comprise a mediator polymer comprising a polymeric material and a metal containing complex, for example a modified poly(vinylpyridine) backbone loaded with poly(bi-imidizyl) Os complexes covalently coupled through a bidentate linkage. Further, the at least one test chemical may be comprised in a single layer, or the test chemical may comprise a plurality of layers, such as one layer having the at least one enzyme and one or more additional layers having one or more additional functions, such as one or more diffusion barriers and/or one or more biocompatibility layers.

The other one of the measurement electrodes 114 may be designed as counter electrode 118. The counter electrode may be a part of the implanted or partially implanted analyte sensor, or may be an individual electrode, which is either implanted or partially implanted or placed somewhere else on the body, e.g. on the skin surface. In Figure 1 for the counter electrode 118 a capacitance representing the electric double layer and a resistance representing the charge transfer resistance is shown. The counter electrode 118 may be configured for performing at least one electrochemical counter reaction and/or configured for balancing a current flow required by the detection reaction at the working electrode 116. In case of the analyte sensor 112 comprises a two electrode system as measurement electrodes 114, the counter electrode 118 may complete the circuit such that charge can flow through an electrochemical cell, also denoted electrochemical system, given by the working electrode 116, the counter electrode 118 and an electrolyte, such as the bodily fluid, and may maintain a constant counter electrode potential, also referred to as a constant reference potential, regardless of current.

Additionally, the analyte sensor 112 may comprise at least one reference electrode 120. The reference electrode 120 may be configured for being a reference for measuring and/or controlling a potential of the working electrode 116. The reference electrode 120 may have a stable and well-known electrode potential. The electrode potential of the reference electrode 120 may preferably be highly stable. One of the electrodes may have several functionalities, as for instance, combined reference and counter electrode, which has both, the function of the reference electrode 120 and counter electrode 118, which means it provides a reference potential and balances the current flow from the working electrode 116.

At least one of the measurement electrodes 114 comprises at least one membrane element 122 having at least one membrane property. In Figure 1, the resistance of the electrolyte between the working electrode 116 and the reference electrode 120 may be given by an electric resistance $R_2$ and the resistance of the electrolyte between the counter electrode 118 and the reference electrode 120 may be given by an electric resistance $R_1$. The resistance $R_2$ may further be dependent on properties of the membrane element 122 denoted with an arrow and reference number of the membrane element at the electric resistance $R_2$. Specifically, the membrane element 122 may be applied to the working electrode 116. The membrane element 122 may be configured for controlling and/or limiting diffusion of the analyte to the working electrode 116. Thus, the membrane element 122 may be configured as diffusion limiting membrane. However, the membrane element 122 may have even more functionalities, such as providing biocompatibility. The membrane element 122 may have further functions such as blocking of leakage of components below the membrane element 122 such as of the enzyme or other components comprised in any one of the at least two measurement electrodes. The membrane element 122 may also be configured as a blocking membrane. The blocking may refer to preventing leakage of inner components of a sensitive layer of the working electrode 116 but not to the analyte. The membrane element 122 may be configured for maintaining of sensor integrity, by for instance keeping the enzyme or redox mediator from leaching, thus degradation of the whole sensor. Independently on the role of the membrane element 122, its altering may be compensated.

The membrane element 122 may comprise at least one polymer. The membrane element 122 may be applied to the working electrode 116 as thin polymer film. For example, the membrane element may be or may comprise Poly-(4-(N-(3-sulfonatopropyl) pyridinium)-co-(4vinyl-pyridine)-co- styrene (5%/90%/5%) or hydrophilic Polyurethane (HP60D20), for example available from Lubrizol®. For example, the membrane element may comprise at least one of the following polymer classes and/or their copolymer: Poly(4 vinyl pyridine), Polymethacrylate, Polyacrylate, Polyvinyl pyrrolidone, Polyvinyl alcohol (PVA), Polyethylene glycol.

The analytical system 110 may be configured for determining the at least one membrane property. Permeability of the membrane element 122 for certain compounds may be proportional to the membrane's swelling degree. The swelling degree may correspond to the degree of water uptake. The swelling degree of the membrane 122 may depend on its hydrophilicity. The membrane's swelling degree may directly affect the amount and/or mobility and, thus, the permeability of the membrane for certain compounds. The conductivity of an electrolyte like water or bodily fluid, such as interstitial fluid is directly linked to so-called total dissolved solids whereby ions, such as H+, OH-, Na+, K+, Cl- and other have the most contribution. Therefore, also the conductivity of the membrane 122 which has taken up water or bodily fluid such as interstitial fluid also is directly linked to the total dissolved solids. The more charge carriers are present and the more mobile they are, the lower is the measured electrical resistance, by otherwise constant conditions, such as e.g. cell geometry. Thus, the electrical resistance, or reversely, electric conductivity of the membrane element 122 may depend on quantity and mobility of ions present in the membrane. The analytical system 110 may be configured for using at least one algorithm configured for determining permeability of the membrane element 122 for a specific analyte, in particular glucose, by evaluating electrical resistance of the membrane element 122. The permeability of the membrane element 122 for a specific analyte $p_{Analyt}$ may be determined by $p_{Analyt} = f*p$, wherein p is the permeability determined via the electrical resistance of the membrane element 122 and f is a conversion factor. The conversion factor may be determined in calibration experiments using known glucose values.

The membrane property, in particular the permeability, may depend on different parameters such as temperature, composition of interstitial fluid, thickness of the membrane element, aging, swelling degree, mechanical stresses and the like. The analytical system 110 may be configured for performing at least one calibration step, wherein effects of the different parameters on the permeability of the membrane element 122 may be determined. For each of the parameters influencing permeability of the membrane element 122 at least one correction factor may be determined by calibration experiments. The analytical system may be configured for determining correction factors for interdependent parameters. The analytical system 110 may be configured for determining permeability of the membrane element 122 considering the at least one correction factor. The analytical system 110 may be configured for in-operando monitoring of permeability, in particular continuously or in short time intervals. Also temperature monitoring is possible. The analytical system 110 may be configured for performing at least one failsafe step in order to enhance reliability of the determining of the analyte concentration.

The analytical system 110 comprises at least one signal generator device 124 configured for generating at least one fast-transient voltage signal. The signal generator device 124 is configured for applying the fast-transient voltage signal to the two measurement electrodes 114.

The fast-transient voltage signal may be at least one arbitrary voltage signal applicable to the at least two measurement electrodes 114 having fast-transient signal flanks, in particular two very steep edges. The fast-transient voltage signal may comprise a square wave form and/or a sine wave form. The fast-transient voltage signal may comprise a non-continuous signal such as a pulse. Specifically, the fast-transient voltage signal may comprise a fast transition square wave. The pulse may have a transient change in the amplitude of the signal from a first value, also denoted baseline value, to a second value, followed by a return to the baseline value or at least approximately to the baseline value. The second value may be a higher or lower value than the baseline value. A pulse duration may be $\leq 50$ $\mu$s, preferably $\leq 20$ $\mu$s, more preferably $\leq 10$ $\mu$s. The duration of the single pulse must be sufficiently long to be able to record its propagation. The duration of the single pulse must be preferentially short, in order to not excite the system electrochemically. The fast-transient voltage signal may be applied during at least one test sequence, for example a time sequence. The fast-transient voltage signal may be applied repeatedly, in particular periodically. The time distance between the cycles must be sufficiently long in order to keep the system at its steady-state. The fast-transient voltage signal may comprise a repeatable cycle, wherein the repeatable cycle comprises at least one signal flank. The signal flank may be a transition of a signal amplitude from low to high signal value or from high to low signal value. The signal flank may be a rising signal flank or a falling signal flank. The signal flank of the fast-transient voltage signal may have a change in signal from the first value of the signal flank to the second value of the signal flank in a microsecond to nanosecond range. The signal flank of the fast-transient voltage signal may have a change in signal from the second value of the signal flank to the first value of the signal flank in a microsecond to nanosecond range. The terms first and second "value" may refer to regions or points of the fast-transient voltage signal, in particular signal amplitude. The first value may be the baseline value. The first value may be a local and/or overall minimum of the fast-transient voltage signal. The first value may be a first plateau of the fast-transient voltage signal. The first value may refer to a time point with no voltage is applied to the measurement electrodes. The first value may be a through or low value of the fast-transient voltage signal. The second value may be a local and/or overall maximum of the fast-transient voltage signal. The second point may be a second plateau of the fast-transient voltage signal, which may be reached during application of the fast-transient voltage signal. The second point may be a peak or high value of the fast-transient voltage signal. The fast-transient voltage signal may have steep edges. Specifically, the fast transition square wave may have a change in signal from the first value of the signal flank to the second value of the signal flank below 50 ns, preferably below 20 ns. The change in signal from the first value of the signal flank to the second value of the signal flank may be even faster and may be only limited by electronics such as by an analog-to-digital-converter. The faster the flank and the sharper the transition to the plateau, the more resolution may be between the ohmic part of the system resistance and the capacitive part of the system capacitance. Without wishing to being bound by theory, the fast-transient voltage signal is so short, in particular ultrashort, that no faradaic currents are generated and that an electrochemical system of the analyte sensor 112 is not disturbed and brought out of equilibrium. The ultrashort voltage signal of the fast-transient voltage signal for determining the membrane property may allow that a measurement signal for determining the analyte concentration can be undisturbed determined. The ultrashort voltage signal may prevent that side reaction occur.

The signal generator device 124 may comprise at least one function generator selected from the group consisting of: at least one square wave generator and at least one sine wave generator. The signal generator device 124 may be part of measurement electronics of the analyte sensor 112 and/or may be connected to the analyte sensor 112 and may be designed as a separate device.

The analytical system 110 is configured for determining the membrane property based on a fast-transient measurement principle. A possible implementation is shown in Figure 1. The unknown resistance of the membrane to be determined is serially connected with a known reference resistor, denoted in Figure 1 R3, with a value roughly matching the range of the unknown resistance. The signal generator device 124 is configured for applying the fast-transient voltage signal at the two serially connected resistances and simultaneously measures the voltage drop at one of the both resistors: either at the reference one, or at the unknown. Knowing the applied voltage and the voltage drop at one of the both resistances, the value of the unknown resistances can be calculated.

The analytical system 110 comprises and/or may be directly connectable to at least one measurement unit 126, in particular at least one microcontroller unit (MCU) or an analog front end (AFE), configured for receiving at least one response signal. The analyte sensor 110 may comprise and/or may be directly connectable to the MCU or AFE. For example, the analyte sensor 110 may comprise sensor contacts 128 via which the analyte sensor 112, in particular the measurement electrodes 114 can be connected to the MCU. The signal generator device 124 may be part of the MCU or may be a separate device. The signal generator device 124 may be configured for applying the fast-transient voltage signal to the measurement electrodes 114. The MCU may comprise at least one digital output, in particular a first digital to analog converter DAC output, denoted "Pulse" in Figure 1, via which the fast-transient voltage signal can be generated and/or applied to the measurement electrodes 114. The fast-transient voltage signal may be applied to at least two measurement electrodes 114 in at least one signal application step. The fast-transient voltage signal may be applied to the working electrode 116.

The response signal may be a measured propagation of the applied fast-transient voltage signal. The response signal may refer to equivalent series resistance of the analyte sensor 112. The MCU or AFE may be configured for determining the voltage at the working electrode 116 during application of the fast-transient voltage signal.

The analyte sensor 112 may comprise and/or may be connected to at least one potentiostat 130 and/or may be part of at least one potentiostat 130, in particular at least one analog or digital potentiostat, configured for determining the concentration of the analyte. Operating principles of potentiostats for continuous monitoring of analytes are generally known to the person skilled in the art. The potentiostat 130 may be configured for generating and/or applying of at least one measurement voltage signal, in particular a polarizing potential or voltage. For example, the potentiostat may be based on a MCU which may comprise at least one second Digital to Analog converter (DAC), denoted DAC in Figure 1, or at least one PWM output, optionally with a low pass filter for generating and/or applying of at least one measurement voltage signal.

The measurement voltage signal may be a voltage signal used for determining the concentration of the analyte. The measurement voltage signal may be different to the fast-transient voltage signal. In particular, the measurement voltage signal may be longer compared to the fast-transient voltage signal. The measurement voltage signal may be a permanent signal, not a pulsed one. The measurement voltage signal may be adjusted from time to time or continuously in order to give the analyte sensor its polarization voltage, preferably, in order to keep the predefined polarization voltage at the analyte sensor. The measurement voltage signal may be a continuous direct current (DC) signal which polarizes the electrochemical cell, and serves as the "motor" for the amperometric measurement of the analyte reducing or oxidizing GOx across the electrochemical cell. The fast-transient voltage signal may be a voltage pulse with high frequency that only characterizes the capacitive and ohmic parts of the electrochemical cell. Therefore, the measurement voltage signal and the fast-transient voltage signal may not influence each other, since they have completely different time domains.

The potentiostat 130 may comprise at least two Analog to Digital channels (ADC) for determining voltage output at the two measurement electrodes. In case of using a reference electrode, the potentiostat 130 may comprise four Analog to Digital channels. The MCU may be configured for regulating the output of its "DAC" in order to get a wanted polarization voltage, for example 50 mV, between the reference electrode 120 and the working electrode 116. The measurement voltage signal may be the output signal of the "DAC". The current flowing through the analyte sensor 112 may be measured on the counter electrode 118 by using an ohmic resistance and at least one first operational amplifier, denoted Amp1 in Figure 1, connected with the counter electrode 118. The output of said first operational amplifier may be connected to a first ADC channel, denoted ADC1 in Figure 1. The reference electrode 120 may be a high-impedance electrode and may control the potential of the potentiostat 130. A second operational amplifier, denoted Amp2 in Figure 1, may be connected to the reference electrode 120 in order to guarantee that no current is flowing out of the reference electrode 120. The potential between the reference electrode 120 and the working electrode 116 may be controlled via a second ADC channel, denoted ADC2 in Figure 1, and a fourth ADC channel, denoted ADC4 in Figure 1, wherein, for example, the second ADC channel may be connected to the output of the second operational amplifier and the fourth ADC channel may be connected to the working electrode 116.

For measuring the response signal to the fast-transient voltage signal the analyte sensor 112 and/or the MCU may comprise further components. For example, the microcontroller unit may comprise two additional capacitors, two additional resistors, one additional ADC channel and the first digital output, as outlined above. One of the additional capacitors, denoted C1 in Figure 1, may be connected to a non-inverting input of the first operational amplifier connected to the counter electrode 118. The other additional capacitor, denoted C2 in Figure 1, may be arranged in series with the first digital output of the MCU. The third ADC channel, denoted ADC3 in Figure 1, may be connected to the working electrode 116 such that the two ADC channels, i.e. the third and the fourth ADC channel, are connected to the working electrode 116. The fourth ADC channel may be connected directly to the working electrode 116. The fast-transient voltage signal may be applied to the working electrode 116 in series with a reference resistance, denoted $R_3$. $R_3$ may be a known reference resistance such as a predetermined reference resistance. The reference resistance may be an average value determined, specifically pre-determined, from a plurality of reference measurements. The reference resistance must reflect the measurement range of the cell. This reference resistance may reflect required measurement tolerances which must be maintained for correct system resistances. The reference resistance may be selected suitable for determining a value to be measured such as the electrical resistance of the membrane element. The fast-transient voltage signal may be determined such as by using the third ADC channel which may be placed in series and between the first digital output and the reference resistor $R_3$. Specifically, before the application of the fast-transient voltage signal an output of the third ADC channel may correspond to the measurement voltage signal. After the application of the fast-transient voltage signal an output of the third ADC channel may correspond to the sum of the measurement voltage signal and the fast-transient voltage signal. The potentiostat 130 may be configured for determining the propagation of the fast-transient voltage signal applied to the working electrode 116. The potentiostat 130 may be configured for determining a change or difference $\Delta V_{ex}$ of the voltage signal at the reference resistor $R_3$ before application of the fast-transient voltage signal and during the application of the fast-transient voltage signal. The potentiostat 130 may be configured for determining a change or difference $\Delta V_{prop}$ of voltage at the working electrode 116 before application of the fast-transient voltage signal and during the application of the fast-transient voltage signal.

The analyte sensor may comprise at least one isolating resistor, denoted $R_4$ configured for isolating the low impedance DAC output, in particular the measurement voltage signal or cell polarization voltage, from the fast transient voltage signal. Without $R_4$ the pulse would be absorbed by the DAC and not the electrochemical cell. The two additional resistors may be arranged in series. A first additional resistor, denoted $R_4$, may be connected with the second DAC and with $R_3$, also denoted second additional resistor. The second additional resistor may be connected to the working electrode 116. The third ADC channel may be arranged between the first additional resistor and the second additional resistor.

The MCU 124 comprising ADC and DAC, wherein the DAC may be replaced by filtered PWM or digital output, depending on the applications site, may be configured for digitally controlling the applied working potential at the analyte sensor 112. The R1 and R2 in the scheme of Figure 1 represent the membrane resistance, which has to be determined. Therefor the "Pulse" output of the MCU generates the fast-transient signal. The amplitude of the pulse is directly measured by the ADC3. The C2 and C1 are acting like short cut during the pulse application, thus the whole amplitude of the pulse is distributed over the resistor R3, which is the reference resistor and the R1-R2 chain. The voltage drop is measured between the reference resistor R3 and the analyte sensor 112 using the ADC4 against ground, thus effectively at the analyte sensor 112. All the other components on the scheme are used for the DC current measurement and are not discussed here.

In order to perform the determining of the membrane property with high accuracy, acquisition of the response signal, in principle, must happen immediately after the fast-transient voltage signal is applied, because of a profile of the fast-transient voltage signal. Once the fast-transient voltage signal is applied at the analyte sensor 112, the analyte sensors' capacitive parts, such as double layer capacitance, are starting to charge. At the very beginning, the capacitive parts can be considered as a short cut, and, thus, corresponding resistive parts are short cut and do not play any role in the voltage drop across the analyte sensor 112. As longer the potential pulse continues, as more the capacitive parts in the analyte sensor 112 may get charged, which may result in an additional voltage drop over these capacitors and, thus, also over the resistive parts so that the measurement may get inaccurate. In order to avoid undesired voltage distribution, as described above, the applied fast transient voltage signal must be as short as possible. Theoretically, the fast-transient voltage signal may be infinitely short. In practice, modern electronics may be sufficiently fast to reach a desired voltage magnitude within few ns. Usually, a limiting factor may be an acquisition speed of measurement electronics of a measurement unit such as of an analog-to-digital-converter (ADC), which is limited. The measurement electronics such as the ADCs as described above may convert an input voltage in digital form and compare it internally with internally generated and digitalized voltages (Successive-Approximation ADC). This process is called conversion. A minimal duration of this process may be determined by resolution and clock of the ADC, and takes, typically, few $\mu$s. Prior to this conversion, the input voltage may be sampled within an ADC channel. This is typically done by charging a small internal capacitor. Therefor the ADC may have corresponding switches: during the sampling, an external voltage to be determined is connected to the internal capacitor of the ADC. Once the capacitor is fully charged, it has the same voltage at its terminals as the input voltage to be determined. After that, the switches disconnect the external voltage and connect the capacitor to the internal converting and comparing unit. A limiting factor during this sampling phase may be the time, which is needed to charge the internal capacitor. In Figure 1, it can be seen, that the "Pulse" output generated voltage is distributed over R3 and R1/R2. The ADC4 input is used to measure the voltage drop at the sensor (R1/R2). In order to charge the ADC4 internal capacitor, the current must flow through the "equivalent output resistor of the Thevenin source present at WE", which is R3 in parallel to R1 + R2. As higher the three resistor are, as longer is the duration of the internal capacitor charging. The sampling time can be configured programmatically, but may not be lower, as needed for the full capacitor charge, otherwise the voltage at the capacitor does not reach the input value and the measurement is then wrong. Thus, the acquisition of the voltage value at the measurement electronics' input may take few microseconds because of the sampling and the conversion. Thus, the recorded voltage drop at the analyte sensor comprises certain error. In principle, the sampling time may be reduced by introducing further components in the schematics, like voltage follower, but this is not an option for a low-cost electronics.

As outlined above, it is impossible to record the voltage immediately after the pulse application. The present invention, in particular, proposes recording of response signals, in particular the voltage drop, at least two times and extrapolation of the recorded values of the response signal towards the time point $t_0$ of the application of the fast-transient voltage signal. The measurement unit 126 may be configured for precisely providing time ticks of voltage acquisition. Still, these two voltage acquisitions may be performed within shortest possible time after the application of the fast-transient voltage, in particular in view of an exponential character of charging of the capacitive parts. Since properties of these capacitive parts may not be well known and/or may also not be stable over time, it may be not possible and/or reliable to perform an exponential fitting. Therefore, the voltage acquisitions may be performed so fast, that there is still sufficiently linear range of an exponent.

The measurement unit 126 is configured for measuring at least two response signals, i.e. the first response signal $U_1$ and the second response signal $U_2$. The measurement unit 126 may be configured for measuring further response signals, e.g. before and/or after and/or between the first and second response signal. However, the first response signal $U_1$ is measured at a first time $t_1$ and the second response signal $U_2$ is measured at a second time $t_2$ with $t_0 \neq t_1 \neq t_2$, wherein the application time $t_0$ precedes the first time $t_1$ and the second time $t_2$. The first time and the second time may be arbitrary time points which fulfill the mentioned requirements. The first time $t_1$ may be in a first time range after the application time $t_0$. The second time $t_2$ may be in a second time range after the first time $t_1$. Lower limits of the first time range and the second time range may be defined by time resolution of at least one measurement unit configured for receiving the first response signal and the second response signal. Upper limits of the first time range and the second time range may be defined by charging characteristics of the capacitive parts of the analyte sensor 112. The voltage pulse may induce flow of capacitive and faraday currents. In order to maintain the sensor integrity, the faraday current flow should be excluded. Therefore, the voltage pulse amplitude and duration must be fitted to the sensor capacitance and the membrane resistance and be as low and short respectively, as possible to avoid inducing the faraday current flow. Considering a sensor with a capacitance <10 nF and $R_{mem}$ < 10 kOhm and the pulse amplitude of 1.5 V, the faraday current starts to flow after roughly 3 $\mu$s. Thus, this duration shall not be exceeded in order to avoid faraday currents. However, the pulse duration may be longer if sensor capacitance is larger and/or $R_{mem}$ is higher. Furthermore, the faraday current may be allowed, once the sensor design considers it. The charging characteristics may be or may comprise charging behavior as a function of time and/or time dependence of charging. The charging characteristics may follow a charging curve Q(t). The charging curve may be an exponential curve. Thus, the measuring of the first response signal and the second response signal may be performed in view of the exponential character of charging of the capacitive parts. The measuring of the first response signal and the second response signal may be performed within shortest possible time after the application of the fast-transient voltage. Since properties of the capacitive parts may not be well known and/or may also not be stable over time, it may be not possible and/or reliable to perform an exponential fitting. Therefore, the voltage acquisitions may be performed so fast, that that the charging curve is still in its linear part. The first time $t_1$ may be in the range from 1 $\mu$s to 5 $\mu$s after the application time $t_0$. The second time $t_2$ may be in the range from 1 $\mu$s to 5 $\mu$s after the first time $t_1$. The measurement unit 126 may be configured for determining the first and second time, in particular with high precision.

The analytical system 110 comprises at least one evaluation device 132. The evaluation device 132 is configured for determining the response signal $U_0$ at the application time $t_0$ by evaluating the first response signal $U_1$ and the second response signal $U_2$. The evaluating may comprise a process of extrapolating and/or deriving the response signal $U_0$ from the measurements of the first response signal $U_1$ and the second response signal $U_2$. Thus, the response signal $U_0$ may not be measured directly but may be evaluated from the first response signal $U_1$ and the second response signal $U_2$. The evaluating may comprise applying at least one fit procedure. The fit procedure may comprise fitting the first response signal $U_1$ and the second response signal $U_2$ by using at least one fit function, in particular a linear fit function $U(t) = b \cdot t + a$ with b being the slope and a the intercept. By using the measured points $(t_1, U_1)$ and $(t_2, U_2)$ the fit parameters b and a may be determined. The determined linear function may be used for extrapolation of the measured first response signal at $t_1$ and the second response signal at $t_2$ towards the time point to of the application of the fast-transient voltage signal for determining $U_0$.

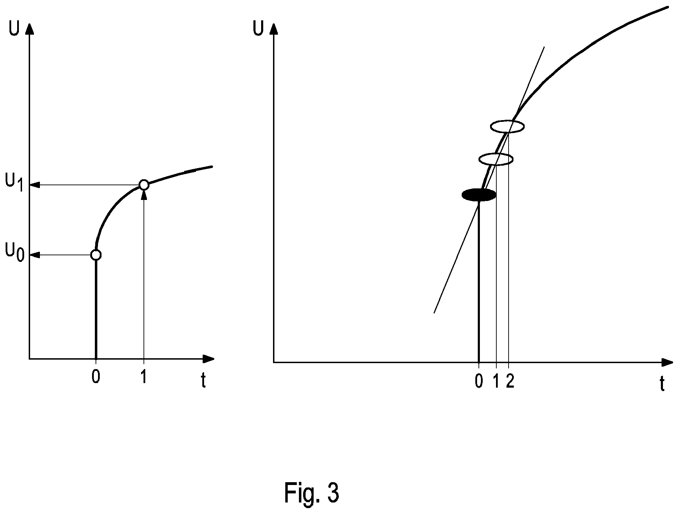

Figure 3, left side, shows schematically the voltage measured at the analyte sensor 112, e.g. by using an infinitely fast voltmeter, as a function of time. Once the voltage pulse is applied at the time point "0", the "at the analyte sensor 112 measured voltage" reaches the values "U0" theoretically infinitely fast. After that the capacitive elements of the analyte sensor 112 start to charge. This leads to inclusion of the charge transfer resistances in the resistances chain and thus to undesired voltage distribution over the whole chain of these resistances. This leads first to the depicted curved voltage increased until the voltage value is reached, which corresponds to voltage drop across the whole analyte sensor 112 including the charge transfer resistances. It is exemplarily shown, that at the time point "1" the voltage "U1" is measured, which partially includes voltage drop at the capacitors or charge transfer resistances, which is undesired.

As outlined above, it is technical impossible to measure the voltage "U0" at the time point "0". Instead, the present invention proposes, measuring at least two voltages at the time points "1" and "2", shown in Figure 3 right side.

Considering, that all measured voltages are lying on one line and the time point "0", "1" and "2" are known, as well as "U$_1$" and "U$_2$", so that it is possible to calculate the value of "U$_0$" which was present at the sensor at the time point "0".

The evaluation device 132 is configured for determining the at least one membrane property by evaluating of the response signal U$_0$. The evaluating of the response signal U$_0$ may comprise determining equivalent series resistance of the electrochemical system of the analyte sensor 112 and determining the at least one membrane property from the equivalent series resistance of the electrochemical system of the analyte sensor 112. The equivalent series resistance of the electrochemical system may be determined by

$$R_1 + R_2 = R_3 \frac{\Delta V_{prop}}{\Delta V_{ex} - \Delta V_{prop}} =$$

$$R_3 \frac{V_{prop,duringPulse} - V_{prop,beforePulse}}{\left(V_{ex,duringPulse} - V_{ex,beforePulse}\right) - \left(V_{prop,duringPulse} - V_{prop,beforePulse}\right)}$$

wherein $v_{prop,beforePulse}$ refers to the voltage at the working electrode 116 before applying the fast-transient voltage signal, $V_{prop,duringPulse}$ refers to the voltage at the working electrode 116 during applying the fast-transient voltage signal, $V_{ex,beforePulse}$ refers to the voltage signal at the reference resistor R$_{ref}$ before applying the fast-transient voltage signal, $V_{ex,duringPulse}$ refers to the voltage signal at the reference resistor R$_3$ during applying the fast-transient voltage signal. Before the application of the fast-transient voltage signal $V_{ex,beforePulse}$ may refer to a voltage at the reference resistor R$_3$ in response to the measurement voltage signal. After the application of the fast-transient voltage signal $V_{ex,duringPulse}$ may refer to the voltage at the reference resistor R$_{ref}$ in response to the measurement voltage signal and due to the propagation of the fast-transient voltage signal.

The analyte sensor 112 may be an in vivo sensor, specifically at least an in vivo continuous glucose sensor. The determining of the membrane property may be performed an in-process control. The determining of the membrane property may be performed during in vivo measurement. The determining of the membrane property may be performed in-operando. Specifically, the determining of the membrane property may be performed during determining of the concentration of the analyte. Additionaly or alternatively, determining of the membrane property may be performed during manufacturing of the analyte. For example, the manufacturing process may comprise at least one calibration, wherein the analyte sensor 112 may be operated with a sample of known analyte concentration.

The technical realization of the measurement setup may be simple and requires only a minimum amount of additional components in addition to a known potentiostat. The determined response signal may not require further processing and may be directly digitalized. The measured response signal may provide absolute values and not relative changes. The determined electrical resistance may be very selective to the membrane property. In particular, the measured electrical resistance may not comprise resistance relating to charge transfer processes of the electrochemical system. Thus, it may be possible to exclude the influences, e.g. of the test chemistry, to the response signal.

Figure 2 shows a flowchart of a method for determining the at least one membrane property of an analyte sensor 112 according to the present invention. The method comprising the following steps:

a) (reference number 134) generating at least one fast-transient voltage signal and applying the fast-transient voltage signal to the measurement electrodes 114 at an application time to;
b) (reference number 136) measuring a first response signal U$_1$ at a first time t$_1$ and a second response signal U$_2$ at a second time t$_2$ with $t_0 \neq t_1 \neq t_2$, wherein the application time to precedes the first time t$_1$ and the second time t$_2$;
c) (reference number 138) determining a response signal U$_0$ at the application time to by evaluating the first response signal U$_1$ and the second response signal U$_2$;
d) (reference number 140) determining the at least one membrane property by evaluating of the response signal U$_0$ at the application time t$_0$.

With respect to description of embodiments of the method reference is made to the description of the analytical system 110 given with respect to Figure 1. The method may be used in a method for determining a concentration of at least one analyte in bodily fluid. The method for determining a concentration of at least one analyte comprises at least one analyte measurement step. In the measurement step at least one measurement value of the concentration of the analyte is determined.

List of reference numbers

**[0127]**

110     analytical system
112     analyte sensor
114     measurement electrode
116     working electrode
118     counter electrode
120     reference electrode
122     membrane element
124     signal generator device
126     measurement unit
128     sensor contacts
130     potentiostat
132     evaluation device
134     generating at least one fast-transient voltage signal
136     measuring response signals $U_1$ and $U_2$
138     determining a response signal $U_0$
140     determining the membrane property

**Claims**

1. A method for determining at least one membrane property of an analyte sensor (112), wherein the analyte sensor (112) comprises at least two measurement electrodes (114), wherein at least one of the measurement electrodes (114) comprises at least one membrane element (122) having at least one membrane property, the method comprising the following steps:

   a) (134) generating at least one fast-transient voltage signal and applying the fast-transient voltage signal to the measurement electrodes (114) at an application time $t_0$;
   b) (136) measuring a first response signal $U_1$ at a first time $t_1$ and a second response signal $U_2$ at a second time $t_2$ with $t_0 \neq t_1 \neq t_2$, wherein the application time $t_0$ precedes the first time $t_1$ and the second time $t_2$;
   c) (138) determining a response signal $U_0$ at the application time $t_0$ by evaluating the first response signal $U_1$ and the second response signal $U_2$;
   d) (140) determining the at least one membrane property by evaluating of the response signal $U_0$ at the application time $t_0$.

2. The method according to claim 1, wherein the evaluating of the response signal $U_0$ in step d) comprises determining equivalent series resistance of the analyte sensor (112) and determining the at least one membrane property from the equivalent series resistance of the analyte sensor (112).

3. The method according to any one of claims 1 or 2, wherein the first time $t_1$ is in the range from 1 $\mu$s to 5 $\mu$s after the application time $t_0$.

4. The method according to any one of claims 1 to 3, wherein the second time $t_2$ is in the range from 1 $\mu$s to 5 $\mu$s after the first time $t_1$.

5. The method according to any one of claims 1 to 4, wherein the analyte sensor (112) is an in vivo sensor.

6. The method according to any one of claims 1 to 5, wherein the method is performed during in vivo measurement.

7. The method according to any one of claims 1 to 6, wherein the method is performed during manufacturing of the analyte sensor (112).

8. The method according to any one of claims 1 to 7, wherein the method comprises at least one failsafe step, wherein the failsafe step is triggered depending on the determined membrane property.

9. The method according to any one of claims 1 to 8, wherein the membrane property is permeability of the membrane element (122).

10. The method according to any one of claims 1 to 9, wherein the fast-transient voltage signal has a square wave form or a sine wave signal form.

11. The method according to any one of claims 1 to 10, wherein the fast-transient voltage signal comprises a non-continuous signal such as a pulse, wherein a pulse duration is $\leq 20\ \mu s$, preferably $\leq 10\ \mu s$.

12. Method for determining a concentration of at least one analyte in bodily fluid using at least one analyte sensor (112), wherein the analyte sensor (112) comprises at least two measurement electrodes (114), wherein at least one of the measurement electrodes (114) comprises at least one membrane element (122) having at least one membrane property, wherein the method comprises determining at least one membrane property of the analyte sensor (112) according to any one of claims 1 to 11, wherein the method comprises at least one analyte measurement step, wherein in the measurement step the concentration of the analyte is determined.

13. A computer program comprising program means for performing the method according to any one of claims 1 to 11 and/or the method according to claim 12 while the computer program is being executed on a computer or on a computer network.

14. An analytical system (110) for determining a concentration of at least one analyte in bodily fluid, wherein the analytical system (110) comprises at least one analyte sensor (112), wherein the analyte sensor (112) comprises at least two measurement electrodes (114), wherein at least one of the measurement electrodes (114) comprises at least one membrane element (122) having at least one membrane property, wherein the analytical system (110) comprises at least one signal generator device (124) configured for generating at least one fast-transient voltage signal, wherein the signal generator device (124) is configured for applying the fast-transient voltage signal to the two measurement electrodes (114), wherein the analytical system (110) comprises at least one measurement unit (126) configured for measuring a first response signal $U_1$ at a first time $t_1$ and a second response signal $U_2$ at a second time $t_2$ with $t_0 \neq t_1 \neq t_2$, wherein the application time to precedes the first time $t_1$ and the second time $t_2$, wherein the analytical system (110) comprises at least one evaluation device (132), wherein the evaluation device (132) is configured for determining a response signal $U_0$ at the application time $t_0$ by evaluating the first response signal $U_1$ and the second response signal $U_2$, wherein the evaluation device (132) is configured for determining the at least one membrane property by evaluating of the response signal $U_0$ at the application time $t_0$.

15. The analytical system according to claim 14, wherein the analytical system (110) is configured for performing the method according to any one of claims 1 to 11 and/or the method according to claim 12.

Fig. 1

Fig. 2

Fig. 3

Europäisches
Patentamt
European
Patent Office
Office européen
des brevets

**EUROPEAN SEARCH REPORT**

Application Number

EP 21 15 9658

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2007/299617 A1 (WILLIS JOHN P [US]) 27 December 2007 (2007-12-27) * abstract; figures 1-25 * * paragraphs [0049], [0104] - [0292] * ----- | 1-15 | INV. A61B5/145 A61B5/1486 A61B5/1473 G01N27/327 A61B5/00 |
| A,D | WO 2019/115687 A1 (ROCHE DIAGNOSTICS GMBH [DE]; HOFFMANN LA ROCHE [CH] ET AL.) 20 June 2019 (2019-06-20) * abstract; figures 1-4b * * page 51, line 20 - page 62, line 23 * ----- | 1-15 | |
| A | US 2015/060302 A1 (MALECHA MICHAEL [GB]) 5 March 2015 (2015-03-05) * abstract; figures 1-9 * * paragraphs [0078] - [0176] * ----- | 1-15 | |

TECHNICAL FIELDS
SEARCHED      (IPC)

A61B
G01N

The present search report has been drawn up for all claims

| Place of search | Date of completion of the search | Examiner |
|---|---|---|
| The Hague | 14 July 2021 | Carta, Riccardo |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 21 15 9658

This annex lists the patent family members relating to the patent documents cited in the above-mentioned European search report.
The members are as contained in the European Patent Office EDP file on
The European Patent Office is in no way liable for these particulars which are merely given for the purpose of information.

14-07-2021

| Patent document cited in search report | | | Publication date | Patent family member(s) | | | Publication date |
|---|---|---|---|---|---|---|---|
| US 2007299617 | A1 | | 27-12-2007 | AU | 2007338662 | A1 | 03-07-2008 |
| | | | | CA | 2657436 | A1 | 03-07-2008 |
| | | | | EP | 2034888 | A2 | 18-03-2009 |
| | | | | US | 2007299617 | A1 | 27-12-2007 |
| | | | | WO | 2008079435 | A2 | 03-07-2008 |
| WO 2019115687 | A1 | | 20-06-2019 | CN | 111448452 | A | 24-07-2020 |
| | | | | EP | 3724648 | A1 | 21-10-2020 |
| | | | | JP | 2021507231 | A | 22-02-2021 |
| | | | | KR | 20200096210 | A | 11-08-2020 |
| | | | | US | 2020225184 | A1 | 16-07-2020 |
| | | | | WO | 2019115687 | A1 | 20-06-2019 |
| US 2015060302 | A1 | | 05-03-2015 | AU | 2014314154 | A1 | 03-03-2016 |
| | | | | CA | 2922780 | A1 | 05-03-2015 |
| | | | | CN | 105492900 | A | 13-04-2016 |
| | | | | EP | 3039415 | A1 | 06-07-2016 |
| | | | | HK | 1227094 | A1 | 13-10-2017 |
| | | | | JP | 6404927 | B2 | 17-10-2018 |
| | | | | JP | 2016529507 | A | 23-09-2016 |
| | | | | KR | 20160046877 | A | 29-04-2016 |
| | | | | RU | 2016110886 | A | 02-10-2017 |
| | | | | TW | 201606301 | A | 16-02-2016 |
| | | | | US | 2015060302 | A1 | 05-03-2015 |
| | | | | WO | 2015028579 | A1 | 05-03-2015 |

EPO FORM P0459

For more details about this annex : see Official Journal of the European Patent Office, No. 12/82

**EP 4 049 586 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20100213079 A1
- WO 2019115687 A1
- EP 20162098 A